# EUROPEAN PATENT SPECIFICATION

(11) **EP 4 447 934 B1**
(45) Date of publication and mention of the grant of the patent: **06.08.2025**
(21) Application number: 23833091.4
(22) Date of filing: 15.12.2023
(51) Int. Cl.: A61K 9/06, A61K 9/00, A61K 9/1272, A61K 9/1274, A61K 31/337

(54) **CARRIER AS A MULTIFUNCTIONAL SYSTEM**
TRÄGER ALS MULTIFUNKTIONSSYSTEM
SUPPORT EN TANT QUE SYSTÈME MULTIFONCTIONNEL

(30) Priority: 15.12.2022 EP 22383223
(43) Date of publication of application: 23.10.2024
(73) Proprietor: Lifesome Therapeutics SL, 28006 Madrid (ES)
(72) Inventor: BOUCHET, Ana Maria, 28006 Madrid (ES); RUYSSCHAERT, Jean Marie, 28006 Madrid (ES); VANDIER, Christophe, 28006 Madrid (ES)
(74) Representative: Isern Patentes y Marcas S.L.
(86) International application number: PCT/EP2023/086117
(87) International publication number: WO 2024/126812

(56) References cited:
- EP-A1- 0 785 773
- WO-A1-2011/101408
- SHARMA DEVENDER ET AL: "An Updated Review On:Liposomes as Drug Delivery System", THE PHARMATUTOR MAGAZINE, vol. 6, no. 2, 2 January 2018 (2018-01-02), pages 50 - 62, XP093041374, ISSN: 2394-6679, DOI: 10.29161/PT.v6.i2.2018.50

## Description

### TECHNICAL FIELD OF THE INVENTION

The invention refers to the medical technical field, particularly, a new transporter (carrier) that combines multiple pharmacological activities, being capable of acting as a carrier of hydrophobic and hydrophilic drugs which confer to the carrier a large diversity of therapeutic activities but also having therapeutic activities by itself and being useful in the treatment and/or in the prevention of hyperproliferative diseases.

### BACKGROUND OF THE INVENTION

The scientific validation of the role of the SK3 channel in *in vitro* cancer cell migration is acquired and is illustrated by several publications in international journals (Potier et al., Mol Cancer Ther 2006, 5, 2946-53 ; Chantome et al., Exp Cell Res 2009, 315, 3620-30 ; Potier et al., Biochem Biophys Res Commun 2010, 397, 42-7; Girault et al., Current Cancer Drug Targets, 2011, 11, 1111-25; Sevrain et al., Med. Chem. Commun., 2012,3, 1471-1478; Sevrain et al., Organic & Biomolecular Chemistry, 2013 Jul 21;11(27):4479-87; Chantome et al., Cancer Research, 2013 Aug 1;73(15):4852-61; Potier-Cartereau et al., Rev Physiol Biochem Pharmacol. 2022; 183:157-176). SK3 channel, is a potassium channel that belongs to SKCa family. The suppression of SK3 reduces cancer cell migration while rescue experiments with SK3 restore cancer cell migration capacity and the enforced expression of SK3 increases the capacity to migrate of cancer cells that do not express the channel. This channel is expressed in some cell types (e.g., brain, smooth muscle) and participates in cell excitability by controlling cellular secretion and muscle contraction. When SK3 is expressed in a cancer cell, it increases twice the capacity of cells to migrate and invade a matrix similar to the physiological extracellular matrix. Thus, the cancer cell seems to hijack the physiological function of SK3 found in excitable cells to promote their ability to migrate. This channel is expressed as a complex in nanodomains rich in cholesterol with the Orai1 calcium channel (Chantome et al., Cancer Research, 2013 Aug 1;73(15):4852-61) and the SigmaR1 (Gueguinou et al., Oncogene. 2017 Jun 22;36(25):3640-3647). This complex in nanodomains rich in cholesterol was not observed in non tumor cells nor on excitable cells and is specific to cancer cells (Gueguinou et al., Biochim Biophys Acta. 2014 Mar 6; pii: S0167-4889(14)00083-4; Potier-Cartereau et al., Rev Physiol Biochem Pharmacol. 2022; 183:157-176). This novel and non-physiological function of SK3 allowed us to hypothesize that the SK3 channel could participate in the formation of metastases. Thus, using mouse models of metastatic breast cancers (orthotopic xenografts), it was found that SK3 channel did not regulate primary tumor development but promotes the development of metastases, mainly the development of bone metastases - this having a direct link between the activation of SK3 by calcium and the high calcium concentrations found in the bone environment (Chantome et al, Cancer Research, 2013 Aug 1; 73 (15) :4852-61). More recently, we found that the expression of SK3 is controlled by the epithelial-to-mesenchymal transition transcription factor Zeb1 which leads to an amplification of Ca²⁺ entry and cancer cellular migration (Figiel et al., Cancers (Basel). 2019 Nov 18;11(11):1814). In addition, Zeb1 and SK3 channel were strongly upregulated by hypoxia (a well-established feature of cancer associated with disease aggressiveness) both in vitro and ex vivo in organotypic cultures of human biopsies (Bery et al., Int J Mol Sci. 2020 Jul 6;21(13):4786). In addition, data on SK3 polymorphism in breast cancer patients reveals that this may increase patient susceptibility to taxane neurotoxicity and that the use of SK3 blockers during taxanes administration may represent an interesting approach for the prevention of this neurotoxicity (Rua et al., Clin Cancer Res; 2018, 24 (21); 5313-20).

The proof of concept of the existence of this new mechanism promoting both in vitro and in vivo migration and cancer progression was also obtained by the identification of a new molecule that inhibits SK3 channel activity, Ohmline (1-O-hexadecyl-2-O-methyl-sn-glycero-3-lactose), the subject of this invention (Girault et al., Curr Cancer Drug Targets. 2011 Nov;11(9):1111-25; Jaffres et al., Pharmacol Ther. 2016 Sep; 165:114-31).

Ohmline belongs to a novel class of alkyl-ether-glycolipids developed to inhibit metastatic processes associated to cancer. The compound targets the plasma membrane and affects specifically K+ channels located in nanodomains with a consequent decrease in membrane potential that decline cancer cell migration (Chantome et al, Cancer Research, 2013 Aug 1; 73 (15) :4852-61). Ohmline was found to specifically reduce the migration of SK3-expressing cancer cells with 50% efficiency at 10 nM. Note that, for concentrations up to 1 µM part of the reduction of cancer cell migration was not attributed to SK3 inhibition. The inhibition of cancer cell migration by Ohmline was observed in all SK3 expressing cells tested including breast cancer MDA-MB-435s and prostate cancer PC3 (Chantome et al, Cancer Research, 2013 Aug 1; 73 (15) :4852-61; Figiel et al., Cancers (Basel). 2019 Nov 18;11(11):1814). In addition, Ohmline was found to inhibit Zeb1 upregulation by TGFβ in normoxic and hypoxic conditions and calcium entries in prostate cancer cells (Figiel et al., Cancers (Basel). 2019 Nov 18;11(11):1814; (Bery et al., Int J Mol Sci. 2020 Jul 6;21(13):4786) and Ca²⁺ entry induced by taxane which was increased in cells expressing short versus long CAG alleles of SK3 (Rua et al., Clin Cancer Res 2018; 24(21); 5313-20). Ohmline can dissociate the SK3 channel forming complex in nanodomains rich in cholesterol through decreased phosphorylation of Akt and modulation of anti-EGFR monoclonal antibodies action in colorectal cancer cells. (Chantome et al, Cancer Research, 2013 Aug 1; 73 (15) :4852-61; Gueguinou et al., Oncotarget. 2016 Jun 14;7(24):36168-36184).

If SK3 channel was found to specifically participate in the development of bone metastases, its inhibitor Ohmline was found to reduce not only bone metastasis development but also lung and lymph node metastases. Ohmline delays the development of bone metastases when SK3-expressing cells were injected in intravenous vein (Chantome et al, Cancer Research, 2013 Aug 1; 73 (15) :4852-61). Interestingly, this lipid was found to displace cholesterol, removing its OH moieties away from their main binding sites, forcing a new rearrangement with other lipid groups (Herrera et al., ACS Omega. 2017 Oct 31;2(10):6361-6370).

Thus, Ohmline and analogs were proposed as new anti-metastatic drugs by Jaffres et al., Pharmacol Ther. 2016 Sep; 165:114-31, which discloses synthetic alkyl lipids, such as Ohmline (1-O-hexadecyl-2-O-methyl-rac-glycero-3-β-lactose), that target cell membranes and to decrease cell migration/invasion, leading to the inhibition of metastasis development.

In addition, the international application WO 2011/101408 A1 entitled "Method for preventing or treating cancer metastasis" , discloses glycerolipid compounds having the chemical formula 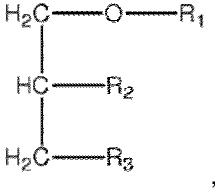 the procedure for synthesizing them and their uses for preventing cancer metastasis. OHM (OHMline), is one of the specific glycerolipids disclosed and used in the assays of said international application, referred there as compound JPH1701, which compound, in accordance to Table 1 of WO 2011/101408 A1, has the formula:

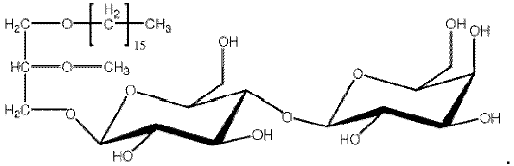

The aim of the invention disclosed in WO 2011/101408 A1 is providing compounds with antimetastatic activity that could be considered true metastasis supressors, which are those compounds that exhibit antimetastatic activity without having any effect on primary tumors. Metastasis supressors are said to act by different mechanisms than tumor suppresors so that, indeed, almost none anticancer agents with proved therapeutic activity against primary tumors possess concomitantly antimetastatic activity. The invention disclosed in WO 2011/101408 A1 provides the use of certain glycerolipid compounds which are also ether lipids, such as Ohmline, for preventing cancer metastasis or for therapeutic treatments against the occurrence of metastasis. Said activity is attributed to the inhibition of the SK3/KCa2.3 channel and is demonstrated by assays where the antimetastatic activity of the glycerolipids (including Ohmline) is proven, also showing that they are specific to the metastatic process and do not affect the primary tumor growth. The assays are carried out by incubating cell cultures with, or administering to model animals, the glycerolipid compounds as such, using DMSO and ethanol as vehicle. WO 2011/101408 A1 teaches the use of pharmaceutical compositions comprising glycerolipid compounds such as Ohmline and a pharmaceutical acceptable carrier, not considering the glycerolipid compounds themselves as possible carriers. The pharmaceutical compositions disclosed in WO 2011/101408 A1 comprise from 0.01% to 99.99% by weight of a glycerolipid of the invention and one or more excipients. The encapsulation in liposomes is one of the delivery systems suggested (although not used in the assays) for the glycerolipids of WO 2011/101408 A1 like Ohmline.

Another important property of the glycerolipids disclosed in WO 2011/101408 A1 is their low cytotoxicity, an effect for which it is necessary, analogously to the anti-metastatic activity, the presence of the glycerol backbone. The low cytotoxicity is an important difference with regard to an analogous compound, edelfosine, which is an alkylglycerophospholipid which is known to have anti-angiogenic and possibly anti-invasive effects (possibly mediated by the presence of a phosphocholine moiety) but also to be highly toxic.

In fact, other phospholipids with an ether moiety in their structure and anticancer activity also show toxicity problems. That is the case of some of the lipids described in European patent application EP0785773A1. The invention disclosed in said application relates to liposomes, which are defined as self-assembled structures comprising one or more lipid bilayers, each of which surrounds an aqueous compartment and comprises two opposing monolayers of amphipathic lipid molecules which comprise a polar (hydrophobic) headgroup region covalently linked to one or two non-polar (hydrophobic) acyl chains so that, in an aqueous medium, lipid molecules are induced to rearrange such that the polar headgroups are oriented towards the aqueous medium while the acyl chains reorient to the interior of the bilayer.

This definition is similar to the definitions that can be found in generic publications about liposomes and other lipid particles, like the review of Sharma *et al.* on liposomes as drug delivery systems (Sharma et al, The Pharma Tutor Magazine, 2018; 6(2): 50-62), where liposomes are said to consist of vesicles composed of bilayers or multilayers that contain or have phospholipids, (which are the main component and are defined as amphiphilic molecules with a hydrophilic head and a hydrophobic tail, usually composed of two acyl chains of 10-24 carbon atoms), and cholesterol, surrounding an aqueous compartment, with a size of 0.01-5.0 µm in diameter and that are formed when phospholipids are hydrated in excess of aqueous medium or aqueous solution. More specifically, it is indicated that the methods for liposome preparation involve four basic stages: a) drying down lipids from organic solvent, b) dispersing the lipid in aqueous media; c) purifying the resultant liposome; d) analyzing the final product. It is also commented that the size determines liposome fate *in vivo,* larger liposomes (diameter greater than 0.1 µm) being cleared from circulation quicker than smaller liposomes, also being more stable in circulation when they have hydrophilic coatings. Other publications (for instance, "The Significant Difference Between Micelles and Liposomes" (https://biologywise.com/difference-between-micelles-liposomes) or describe liposomes in comparison with micelles (lipid nanoparticles with only a layer of amphipathic/amphiphilic molecules), liposomes being larger and with a higher capacity of being loaded with drugs, remarking that their formation is increased beyond a particular temperature (which is known as transition temperature in the case of liposomes and commenting that micelles are mainly formed by surfactant molecules like detergents, emulsifiers, various wetting agents as well as certain co-polymers, while liposomes are formed by phospholipid molecules along with cholesterol. In other divulgation publications, like the web section entitled "Lipids Aggregates in Water - Micelles and Liposomes" (https://bio.libretexts.org/Bookshelves/Biochemistry/Fundamentals_of_Biochemistry_(Jakubo wski_and_Flatt)/01%3A_Unit_I-Structure_and_Catalysis/10%3A_Lipids/10.02%3A_Lipids_Aggregates_in_Water_-Micelles_and_Liposomes) remark the importance of the number of non-polar chains in the structure formed by amphiphilic molecules in water, specifying that some single chain amphiphilic molecules, when added to water, form a monolayer on the surface of the water and, when exceed their solubility, form micelles, while double-chain amphiphiles, in contrast, form bilayers instead of micelles, liposomes being those spherical bilayers that enclose an aqueous compartment.

The liposomes disclosed in European patent application EP0785773A1 in particular comprise a lipid bilayer which is composed of at least two lipids: a lipid with a derivatized head group and a glycerolipid which contains an ether moiety and a backbone similar to that of the glycerolipids of WO 2011/101408 but which differs from said glycerolipids in that it is also a phospholipid, since the radical R3 linked to the carbon located in the other end with regard to the carbon linked to the ether group must be a radical with the formula R3 = R5-P(O)2-O-R6) and, therefore, a phospholipid. The head group derivatized lipid is preferably a phosphatidylethanolamine-dicarboxylic acid. In a preferred embodiment, the liposome lipid bilayer contains an ether-lipid and a lipid with a derivatized head group and a sterol (preferably cholesterol) and a neutral lipid (preferably, phosphatidylcholine). An additional bioactive agent can be present in the liposome. The headgroup-derivatized lipid is included because it can buffer the toxicity of the ether lipid when present in a liposomal bilayer and is said to be, generally, an amphipathic lipid comprising one or more hydrophobic acyl chains and a polar headgroup to which a chemical moiety. Such a composition gives rise to liposomes unilamellar or multilamellar, of less than 200 nm, preferably greater of 50 nm. The liposomes are for the use in the treatment of cancer and inflammatory diseases.

Ohmline, in turn, has already passed the chemical optimization phase, as the optimization of its synthesis has been performed (Alkyl ether lipids, ion channels and lipid raft reorganization in cancer therapy. Jaffrès PA, Gajate C, Bouchet AM, Couthon-Gourvès H, Chantôme A, Potier-Cartereau M, Besson P, Bougnoux P, Mollinedo F, Vandier C.Pharmacol Ther. 2016 Sep; 165:114-31. doi: 10.1016/j.pharmthera.2016.06.003. Epub 2016 Jun 8). Ohmline has a good tolerance and an apparent safety in rodents (e.g., no observable tissue damage by a pathologist). The preclinical studies performed *in vitro* have shown no adverse biological activity.

The potential toxicity and genotoxicity of Ohmline was examined by using Ames and micronuclei tests. Ames tests revealed no mutagenic potential and micronuclei test showed only a weak positive effect at 100 µM. These finding indicated no toxicity or genotoxicity of Ohmline at *in vitro* concentrations lower than 10 µM. A low in vitro toxicity was also observed in cancer cell lines MDA-MB-231, HCT116, PC3, HaCaT and fibroblasts: 70% to 100% of cells were still alive with 25 µM Ohmline for 48h compared to almost 0% with Edelfosine (structure close to Ohmline but with a phosphocholine in sn3 instead of the lactose for Ohmline) in same conditions (ImPACcell-Rennes) (Girault et al., Curr Cancer Drug Targets. 2011 Nov; 11(9):1111-25; Jaffres et al., Pharmacol Ther. 2016 Sep; 165:114-31 ; Potier et al., Br J Pharmacol. 2011 Jan;162(2):464-79).

Finally, preliminary pharmacokinetic studies have been conducted and have found that Ohmline was incorporated in the tissues tested (stomach, intestine, kidney, liver, heart and brain) and present in brain still two weeks after the end of its administration. In addition, it was observed the incorporation of Ohmline in primary tumors and bone metastasis tissues (Chantome et al, Cancer Research, 2013 Aug 1;73 (15) :4852-61).

Currently there is no specific treatment against the development of metastases and no treatment for the prevention of metastases. Only conventional treatments as radiotherapy, chemotherapy or hormone therapy (for some cancers) are proposed to treat metastases. Palliative treatments against pain, or against bone resorption (in cases of bone metastases) are also proposed.

However, several clinical trials for the treatment of metastases of prostate cancer resistant to castration have seen emerged, with modest extensions of the median survival (months). A Phase III clinical trial shows that the use of Denosumab - anti-RANKL developed by Amgen Inc. - delays 4.2 months the bone metastasis free survival, with no effect on overall survival (Smith et al. Lancet, 2012; 379:39-46). Denosumab does not target cancer cell but targets bone cells by preventing their lysis induced by the development of metastasis. The anti-RANKL Ab, which might be similar to conventional non-targeted chemotherapy as it does not target a specific marker of tumor cell, appears thus complementary to the anti-SK3 treatment, but with an estimated cost of treatment much more superior.

Hence, there is thus still a need in the prior art for new treatment against the development of metastases and treatment for the prevention of metastases, targeting the SKCa channels, particularly, SK3 channels, having a lower cost with respect to the conventional non-targeted chemotherapy and minimizing the effect on other target and undesirable effects. Preferably, the product designed for such purpose could be also useful in the treatment of other diseases, such as microbial infections.

### SUMMARY OF THE INVENTION

The main objective of the present invention is to develop a new transporter (carrier) that combines multiple pharmacological activities, having a dual capability: being capable of acting as a carrier of compounds with pharmacological action (the compounds commonly known as drugs) but also having therapeutic activities by itself.

To reach this objective the present inventors have designed and built a transporter (Ohmline) which is already pharmacologically active by itself. The structure of the transporter (or carrier) allows the insertion of hydrophobic and hydrophilic drugs and confer to the same transporter (carrier) a large diversity of therapeutic properties. The polyvalent activity of the present transporter (carrier) is a major breakthrough over the prior art.

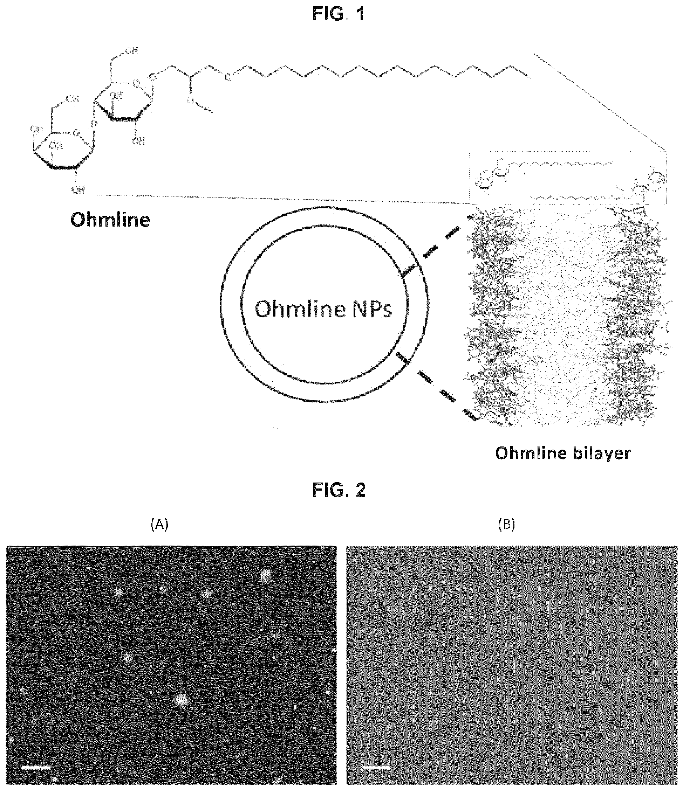

And this has been achieved thanks to having been found that Ohmline, when present in a polar solution like an aqueous solution, forms liposomes spontaneously by themselves, without the need of having in the same solution any of the compounds that are known to form liposomes such as phospholipids or any other amphipathic compound with a polar head and a tail with two apolar chains. This is a surprising finding, since Ohmline, contrary to the liposome forming lipids of previous publications such as those of European patent application EP0785773A1 (which lipids are a phospholipid and another lipid with a derivatized head group which, preferably, is also a derivatized phospholipid and, preferably, are accompanied by a sterol like cholesterol and a neutral lipid like phosphoethanolamine) does not exhibit the structure characteristic of the compounds which forms liposomes in polar solution. In accordance with its structure, Ohmline would have been expected to form (if any) lipid nanoparticles of the kind of micelles, instead of liposomes. However, as it can be seen represented schematically in FIG. 1, Ohmline is capable of forming liposomes where the sugar molecules are exposed at the surface and also at the liposome core defined by the lipid bilayer.

Such property is also surprising for one skilled in the art having knowledge of international patent application WO2011/101408 A1, the document where the Ohmline structure and its anti-metastatic activity were disclosed, since Ohmline activity is assayed in said application using the compound as such, without being assembled forming any structure; and the possibility of forming liposomes, or any other nanoparticle, is not mentioned or suggested in such document.

However, surprisingly, providing a mixture comprising an aqueous buffer, Ohmline in the suitable concentration and, if desired, another compound (preferably one anticancer agent) , and stirring the mixture at a temperature higher than 40ºC (Ohmline transition temperature), gives rise to the formation of liposomes with Ohmline in the lipid bilayer, so that the present invention discloses, among other aspects, a process for preparing Ohmline liposomes. A specific case of such process can be a method of preparing Ohmline liposomes carrying another anticancer agent or antimicrobial agent or any other drug (which will be in the core if it is hydrophilic and in the lipid bilayer if it is hydrophobic). Additionally, if Ohmline concentration is high (equal or, preferably, higher than 3 mg/ml), gel structures are formed, wherein Ohmline molecules are in the form of nanotubes.

The fact than Ohmline self-assembles giving liposomes when the temperature has exceeded its transition temperature (40ºC-44ºC, a temperature range which is specific for Ohmline) allows to have nanoparticles that can be used as carriers of other drugs or compounds with pharmacological action, either hydrophilic compounds (that could be carried encapsulated in the core of the liposome) or hydrophobic (included in the lipid bilayer), with the advantages of liposomes, such as larger capability than micelles for being loaded with other compounds and their easy delivering of cargos to cells by fusing themselves with the cell membranes. Notwithstanding, Ohmline nanoparticles are highly easy to fusion with cell membranes, which facilitates its entry into cells and the release of any cargo in the cell cytoplasm. Moreover, as commented above, when Ohmline concentration exceeds a threshold, Ohmline molecules also allow to form gels wherein the glycerolipid molecules form lipid nanotubes; as shown below in the Examples. Ohmline nanotubes, *in vitro,* form a sustained-release depot, which may also serve as a delivery system for cancer *in situ* treatment. As can be understood from the mentioned *in vitro* assay, by dilution, Ohmline nanotubes become nanoparticles which will release Ohmline, and any encapsulated drug, autonomously, like self-release. Thus, Ohmline might be in the form of two different, but reversible, structural arrangements (gel of nanotubes or liposomes), what may be useful for its delivery.

The particular structure of Ohmline liposomes, which exhibit a surface made of sugars (understanding as such monosaccharides and disaccharides which, in the present case, are specifically lactose moieties), confers them an additional advantage, because it helps to prevent liposomes being cleared from circulation and to allow them to be directed to and arrive at lecithin receptors, which are specifically expressed in tumor and/or inflammation environments.

Moreover, Ohmline liposomes are formed by a compound that has pharmacological effect by itself, so that Ohmline liposomes have the dual capacity of having therapeutic activity by themselves and being able to act as carriers of other drugs. In that sense, Ohmline liposomes have the advantage over the liposomes of EP0785773A1 of having the possibility of having their basic structure being formed by molecules of only one compound, Ohmline. It is another remarkable and advantageous property the low cytotoxicity of Ohmline, while EP0785773A1 acknowledges the toxicity of the anticancer glycerolipids that are one of the components of the liposomes of EP0785773A1 and presents the aim of buffering such toxicity as the main object of the invention disclosed in said European patent application.

It is also remarkable that the assays carried out with Ohmline liposomes have shown that Ohmline administered as nanoparticles, is able to reduce primary tumor size. This is another advantageous and surprising effect, especially in the light of the content of WO 2011/101408 A1, where assays are shown demonstrating that Ohmline, administered as a monomer, does not affect primary tumors. Besides, its metastatic effect not only remains, but it is even increased.

And, additionally, the present inventors have found that Ohmline, in the form of liposomes or gels, can be also used in the treatment of microbial infections, again taking advantage of Ohmline dual capacity of acting as carrier of drugs (antimicrobial agents being preferred in this case) and, also, of acting as an antimicrobial agent by itself, as it is shown in Examples set forth in the present application, where an Ohmline hydrogel depot is shown to exhibit antibacterial activity. This activity appears to be related to the fact that Ohmline molecules targets specifically K+ channels essential for bacterial cellular homeostasis and communication, not only for quorum sensing but also for biofilm formation, biofilm formation having been suggested to account for more than 65% of all infections, *Acinetobacter baumannii, Klebsiella pneumoniae, Staphylococcus aureus, Staphylococcus epidermidis, Pseudomonas aeruginosa* and *Escherichia coli* being considered the most resistant bacteria for treatment due to biofilm formation. In this sense, the fact that Ohmline organized structures expose sugars on their surface, makes them able to bind lectin receptor and to use them for anti-adhesion therapy against bacteria forming biofilms, as lectins have a known role in establishing and holding biofilms together and knock-out or inhibition of biofilm-mediating lectins leads to disruption of biofilm integrity (Inhülsen et al., Microbiologyopen 2012, 1(2), 225-242). Additionally, as commented above, the modulation of the biophysical behaviour of Ohmline turns this material into an outstanding tool for precision medicine to deliver personalized treatment, where two or more antibacterial agents (or antimicrobial agents in general) are necessary in the synergism pursuit.

As used herein, an antimicrobial agent is a compound or a composition which can kill a microorganism, reduce or impede its growth, reduce or impede its entry into cells or its dissemination in a subject or reduce or impede the formation of structures useful for organ colonization such as biofilms. The microorganism can be a bacterium, a protozoon, a microscopic fungus (such as a yeast) or even a virus, and the subject can be any animal (including invertebrates and vertebrates, humans being a possible example) or any plant.

It must be also noted that any references in the description to methods of treatment refer to the compounds, pharmaceutical compositions and medicaments of the present invention for use in a method for treatment of the human or animal body by therapy.

The invention is set out in the amended set of claims.

Consequently, a first object of the present invention refers to a liposome comprising a core and at least one lipid bilayer which comprises a glycerolipid of formula (I): 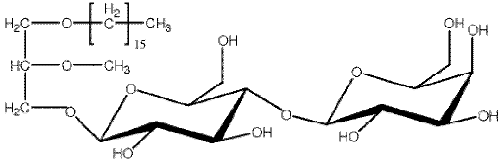
wherein the core comprises a hydrophilic anticancer agent or antimicrobial agent or any other hydrophilic drug and/or
the lipid bilayer comprises a hydrophobic anticancer agent or antimicrobial agent or any other hydrophilic drug.

Preferably, the core comprises a hydrophilic anticancer agent and/or the lipid bilayer comprises a hydrophobic anticancer agent.

As explained above, the lipid bilayer can lack phospholipids or any other amphipathic lipid with a polar head and a double-chained apolar tail. But it can comprise a hydrophobic compound such as an anticancer agent, antimicrobial agent or any other hydrophobic drug.

In another embodiment, the lipid bilayer is composed of the glycerolipid of formula (I). That might be the case where no anticancer agent, no antimicrobial agent and no other drug different from Ohmline itself is comprised in the lipid bilayer.

Thus, in one embodiment, the lipid bilayer consists of molecules of the glycerolipid of formula (I) and the core comprises a hydrophilic anticancer agent.

In an embodiment, the liposome comprises an anticancer agent and the anticancer agent is selected from the list consisting of: aromatase inhibitors, anti-estrogen, anti-androgen, a gonadorelin agonists, topoisomerase 1 inhibitors, topoisomerase 2 inhibitors, microtubule active agents, alkylating agents, anthracyclines, corticosteroids, IMiDs, protease inhibitors, IGF-1 inhibitors, CD40 antibodies, Smac mimetics, FGF3 modulators, mTOR inhibitors, HDAC inhibitors, IKK inhibitors, P38MAPK inhibitors, HSP90 inhibitors, akt inhibitors, antineoplastic agents, antimetabolites, platinium containing compounds, lipid- or protein kinase-targeting agents, protein- or lipid phosphatase-targeting agents, anti-angiogentic agents, agents that induce cell differentiation, bradykinin 1 receptor antagonists, angiotensin II antagonists, cyclooxygenase inhibitors, heparanase inhibitors, lymphokine inhibitors, cytokine inhibitors, bisphosphanates, anti-apoptotic pathway inhibitors, apoptotic pathway agonists, PPAR agonists, inhibitors of Ras isoforms, telomerase inhibitors, protease inhibitors, metalloproteinase inhibitors and aminopeptidase inhibitors, cytostatic agent, cytotoxic agent, taxane, antitumor antibiotic, topoisomerase II inhibitor, topoisomerase I inhibitor, tubulin interacting agent, antibodies, antiangiogenics, COX-2 inhibitors, hormonal agent, thymidilate synthase inhibitor, anti-metabolite, alkylating agent, farnesyl protein transferase inhibitor, signal transduction inhibitor, EGFR kinase inhibitor, antibody to EGFR, C-abl kinase inhibitor, hormonal therapy combination, aromatase inhibitor and combinations thereof. Preferably, the anticancer agent is a taxane selected from the list consisting of docetaxel, paclitaxel, tesetaxel, cabazitaxel.

A second object of the invention refers to a gel of the glycerolipid of formula (I) wherein the glycerolipid molecules form lipid nanotubes.

A third object of the invention refers to pharmaceutical compositions comprising said liposome or gel and at least a pharmaceutical acceptable excipient or/and one adjuvant. In an embodiment, the stabilizer or the surfactant is an anionic/cationic lipid and/or the stabilizer and the surfactant are in a concentration in the composition from 5-45 % w/w, to the total weight of the composition. In another embodiment, combinable with the previous one, the composition is a vaccine.

Another object of the invention is the liposome of the first object of the invention or the gel of the second object of the invention for use as a DNA, siRNA, or RNA, antibodies or nanobodies carrier.

Also an object of the invention is a liposome of the first object, a gel of the second object or a pharmaceutical composition of the third object for use as a medicament.

In a preferred embodiment, the liposome or the gel comprises an antimicrobial agent and the use thereof is in the treatment of a bacterial, viral or a fungal infection. In a particular embodiment, the infection is a biofilm-associated bacterial infection. For instance, the infection can be an infection caused either by a Gram(-) or a Gram(+) bacterial species, such as *Escherichia coli* or *Staphylococcus aureus* or also *Acinetobacter baumannii, Klebsiella pneumoniae, Staphylococcus epidermidis* or *Pseudomonas aeruginosa.,*

In a preferred embodiment, the liposome, the gel or the pharmaceutical composition are for use in the treatment or prevention of cancer and/or in the prevention or reduction of cancer metastasis, wherein the liposome or gel comprise an anticancer agent. In a particular embodiment of that previous one, the use is for primary tumor growth inhibition or reduction. In another particular embodiment, the cancer is selected form the list consisting of: breast cancer, head and neck cancer, stomach cancer, prostate cancer, non-small-cell lung cancer, leukaemia, skin cancer, melanoma, liver cancer, ovarian cancer, pancreatic cancer, lung cancer, kidney cancer, colon cancer, brain cancer, selected from gliomas and glioblastoma, thereof.

Additional objects of the invention refer to a process for the preparation of a liposome and a process for the preparation of a gel comprising the glycerolipid of formula (I), wherein the formation of one or the other structure is dependent on the Ohmline concentration. The process for the preparation of the liposome comprises at least the steps of: (i) providing a mixture which comprises an aqueous buffer, at least one anticancer agent, antimicrobial agent or any other drug and the glycerolipid of formula(I), wherein the concentration of the glycerolipid of formula (I) in the mixture is equal or lower than 3 mg/ml; (ii) stirring the mixture at a temperature higher than 40ºC; wherein, when the anticancer or antimicrobial agent or the other drug is a hydrophobic compound, the anticancer or antimicrobial agent or the other drug is added to the mixture with the glycerolipid of formula (I) obtained in the previous step and wherein, when the anticancer or antimicrobial agent or the other drug is a hydrophilic compound, the anticancer or antimicrobial agent or the other added drug is incorporated in the aqueous buffer. In an embodiment, the hydrophobic anticancer or antimicrobial agent or drug of step i) was previously dried before mixed with the buffer. In another embodiment, combinable with the previous one, the process further comprises the step (iii), which comprises treating the mixture obtained in step (ii) by extrusion or sonication.

The process for the preparation of the gel, in turn, comprises at least the steps of: (i) providing a mixture which comprises an aqueous buffer, at least one anticancer or antimicrobial agent or any other drug and the glycerolipid of formula(I), wherein the concentration of the glycerolipid of formula (I) in the mixture is higher than 3 mg/ml; (ii) stirring the mixture at a temperature higher than 40ºC; wherein, when the anticancer or antimicrobial agent or the added other drug is a hydrophobic compound, the anticancer or antimicrobial agent or the other added drug is added to the mixture with the glycerolipid of formula (I) obtained in the previous step and wherein, when the anticancer or antimicrobial agent or the other added drug is a hydrophilic compound, the anticancer or antimicrobial agent or the other drug is incorporated in the aqueous buffer.

### BRIEF DESCRIPTION OF THE FIGURES

FIG. 1. Schematic representation of Ohmline bilayer organization, exposing sugar molecules to the surface and to the core, while the acyl chains are in the interior part of the bilayer itself. The representation exemplifies the molecular assembly that would be possible to achieve a bilayer from a single acyl chain molecule like Ohmline. Two individual Ohmline molecules can be seen over the bilayer, each of them placed in their expected arrangement according to the layer where they are situated. The figure also shows a magnified version of the same representation of Ohmline formula, with the sugar cycles simplified as simple hexagonal cycles with all members of a same cycle projected on the same plane
FIG. 2: Fluorescence microscopy Images of Ohmline MLV 1mg/ml labelled with the hydrophobic probe BODIPY 493/503 (A) and Phase contrast (B). Scale bar in white = 5 µm. Ohmline multilamellar vesicles result in the visible size range between 500 nm to 2,5 µm.
FIG. 3: Transmission electron microscopy images of Ohmline LUVs prepared by extrusion (A) or sonication (B) at concentration of 100 µM, and 20 µM respectively.
FIG. 4: Phase contrast and fluorescence microscopy images of CF encapsulated in Ohmline MLV. (λex=492nm, λem=517nm). (A) Phase contrast, Fluorescence and Merge field respectively. White Scale bar= 2.5 µm (B) Phase contrast and Fluorescence respectively. Scale bar = 10 µm.
FIG. 5: Optical Microscopy Images of Ohmline MLV labelled with di4ANNEPDHQ. λ excit= 488 nm λ emiss= 520 to 700 nm.
FIG. 6: Sequence in time: figure 6. A=time 0, figure 5.B=time after 0 and figure 5.C=ended. In all the sequences the left tube contains the methanol solution probe as a control of complete miscibility with water. The gel phase of Ohmline remains stable and immiscible in water like a depot.
FIG. 7: The FIG. 7.A correspond to 48 hrs. after the injection of Ohmline gel described in FIG. 6. In all the sequences the left tube contains the control of methanol solution probe miscible in water. The FIG. 7.B correspond to 72 hrs. after the injection where the depot was already cleared in liposomes containing probe.
FIG. 8: Electron-Microscopy of Ohmline 10 mg/ml after heat (over 40°C) vortex cycles for the homogenization. Some milliliters were deposited on the grid for EM measurement and stained with Uranyl acetate (A) Black Scale bar= 100 nm (B) Black Scale bar= 200 nm.
FIG. 9: Sted Microscopy Images of Ohmline hydrogel 4 mg/ml with tetramethylcarbocyanine coencapsulated in the glycerolipid structure. (λex=550 nm, λem=567nm). White scale bar on the images = 2 µm.

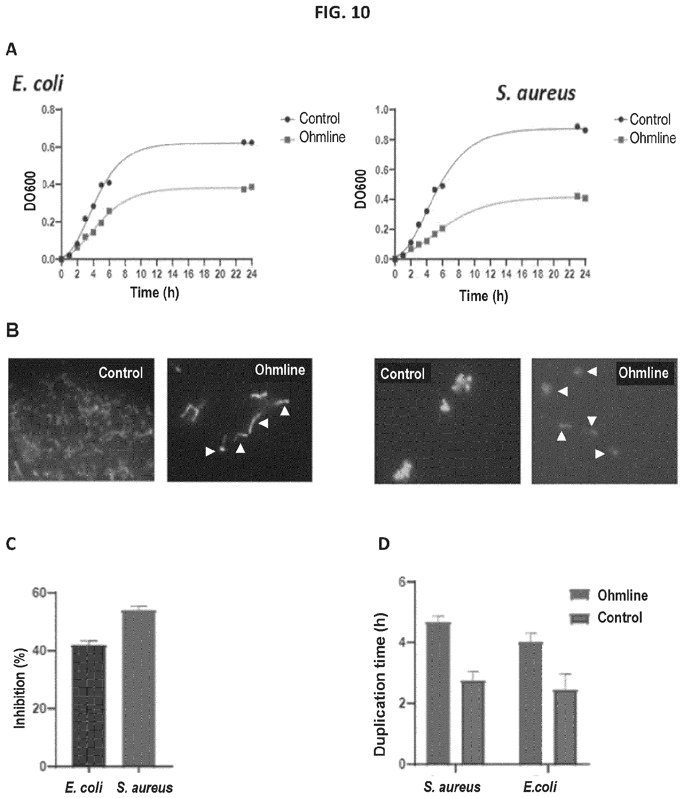

FIG. 10: FIG. 10 A shows bacterial growth curves of *Escherichia coli* (graph on the left) and *Staphylococcus aureus* (graph on the right) representing control (data indicated with circles) and Ohmline-treated bacteria (data indicated with squares). FIG 10 B Fluorescence microscopy images of *E*. *coli* (pair of images on the left) and *S. aureus* (pair of images on the right) showing the internalization of propidium iodide (in red in the original, indicated with arrows in the adapted version) due to the Ohmline treatments. FIG. 10 C is a bar chart showing the percentage of inhibition of bacterial growth due to Ohmline treatment (*E*. *coli:* bar on the left; *S. aureus:* bar on the right), calculated with regard to the value obtained for the control. FIG 10 D: Effect on duplication time of the bacteria indicated under the X-axis due to Ohmline treatment (Ohmline: left bar of each pair; control: right bar of each pair).

### DETAILED DESCRIPTION OF THE INVENTION

The present invention takes advantage of the concept of the inhibition of the activity of a plasma membrane protein; therefore, this does not require internalization of the therapeutic molecule in cancer cells, thereby minimizing the effect on other target and undesirable effects.

Ohmline and its derivatives are known in the prior art as anti-metastatic molecules targeting the SK3 channel.

Ohmline and its derivatives are lipid molecules, retained at the plasma membrane having a simple chemical formula.

Ohmline has a glyco-glycero-ether lipid of chemical formula (1-O-hexadecyl-2-O-methyl-sn-glycero-3-lactose), so that it contains sugars in its structure: two linked monosaccharides (glucose and galactose) which form the disaccharide lactose. Its synthesis can be readily performed using standard organic chemistry protocols. Ohmline demonstrate no acute toxicity in small animals at 15 mg/Kg, up to 4 months treatment. Ohmline is the first specific inhibitor of the K+ channel SK3: its potency was greater for SK3 channel than for SK1 channel; it did not affect IKCa/SK4 channel and only slightly but not significantly affected SK2 channel. This is the first SKCa channel blocker that can be used to discriminate between SK2 and SK1/SK3 channels. This compound, which acts with an IC₅₀ of 300 nM, did not displace apamin (a bee venom extract peptide, a SKCa channel blocker that do not discriminate between SK1, SK2 and SK3 channel) from SKCa channels and had no effect on non-specific edelfosine (alkyl-lipid having a phosphocholine in sn3 glycerol instead of the lactose for Ohmline) targets such as protein kinase C (PKC), receptors for platelet activating factor (PAF) and lysophosphatidic acid (LPA), as well as non-cancerous cells. (Girault et al., Curr Cancer Drug Targets 2011 Nov;11(9):1111-25).

The mechanism of action of Ohmline is different compared to other ion channel blockers: it is not acting as a pore blocker like apamin and like most of ion channel modulators but block SK3 channel activation following its incorporation in plasma membrane ensuing in the inner cellular membranes. Thus, this lipid can be used to decrease the activity of various SK3 channels: apamin-sensitive and apamin-insensitive SK3 channels (an embryonic SK3 channel not sensitive to apamin has been discovered) as well as potential SK3 protein that does not form ion channel (this was found for some potassium protein, eag1, in cancer cells but this remain to be explored for SK3 protein) (Downie et al., J Biol Chem, 2008; 283(52):36234-40).

Moreover, Ohmline molecules, as monomers, target specifically K+ channels essential for bacterial cellular homeostasis and communication (quorum sensing/biofilms). Irrespective of the metabolic state of the bacteria, bacterial K+ channels maintain a critical role in membrane potential modulation, intercellular and interspecies communication, and memory formation (Prindle et al., Nature 2015, 527(7576), 59-63). Membrane potential is essential for sub-second functions such as motility and cellular respiration, but also important for minute-to-hour time scale processes such as cell division and communication between cells. Targeting K+ channels is therefore the key to disrupt bacteria survival, interaction, adaptation and communication in a variety of environmental conditions (Samuel et al., Trends in Microbiology 2021; 29(10), 894-907). Previous studies suggested that biofilm-associated infections account for more than 65% of all infections (Wang et al., Biofilms and Microbiomes 2023, 9(1), 63), a biofilm being defined as a structured consortium attached on a living or inert surface formed by microbial cells sticked to each other and surrounded by a self-produced extracellular polymeric matrix (Wu et al., International Journal of Oral Science, 2014; 7:1-7). Interestingly, a potassium channel deleted-strain revealed a defect in biofilm development emphasizing the relevance of this membrane protein as a target for antimicrobial system (Diggle et al., PloS one 2013; 8(5):e60993).

The inventors have demonstrated that Ohmline can adopt a nanoliposomal structure when it is dispersed in aqueous medium. This recent observation drastically changes the status of Ohmline which should not only be considered as a drug but also a drug transporter. To the best of the inventors' knowledge this duality has never been demonstrated so far.

Along the last years the use of lipid nanoparticles technology in cancer therapy has improved exponentially. Due to their reduced size, lipid nanoparticles decorated with specific antibodies or any proteins able to recognize specific targets accumulate in tumors, with limited effect on other healthy parts of the organism, have the capacity to increase the therapeutic efficacy and to reduce the main adverse effects caused by chemotherapy. 19 lipid nanoparticles commercial versions have been approved for therapy and another 17 in the clinic (Allen and Cullis, Adv. Drug Rev. 2013; 65:36-48; Liu et al., Int. J. Nanomedicine, 2013; 8:3309-3319).

Hence, the present invention provides liposomes and gels which combine an anti-metastatic and anticancer activity of Ohmline, with its capacity to transport any kind of drugs. This unique property is related to the unique capacity of Ohmline to adopt lamellar phase in different nanostructures.

Besides, Ohmlime liposomes and gels of the present invention also show antimicrobial activity by themselves, additionally to the possibility of acting as carriers of other antimicrobial agents. Thus, Ohmline, as an organized structure, can be used for anti-adhesion therapy (AAT) and for antimicrobial drug delivery. Without wishing to be bound by any theory, ATT can be attributed to the fact that Ohmline nanoparticles or gels of nanotubes expose sugars at their surfaces (see, for instance, FIG. 1), thus being able to bind lectin receptors and impeding microbial adhesion. That is consistent with previous findings which indicate that lectins have a role in establishing and holding biofilms together (Diggle et al., Environmental Microbiology 2006, 8(6), 1095-1104).and the knock-out or inhibition or biofilm-mediating lectins leads to disruption of biofilm integrity (Inhüsen et al., Microbiologyopen 2012; 1(2): 225-242; Tielker et al., Microbiology 2005; 151(5): 1313-1323). Moreover, the modulation of the biophysical behaviour of Ohmline turns this material into an outstanding tool for precision medicine to deliver personalized treatment, where two or more antibacterial are necessary in the synergism pursuit.

All the above are advantageous and surprising effects of the structures formed by Ohmline.

For most nanoparticles it is the associated drug that confers its specific activity. For instance, liposome have been used successfully both in vitro and in vivo, but the lipid is just a transporter and does not possess a specific pharmacological activity.

Surprisingly, the inventors have found that liposomes and gels comprising Ohmline are excellent carriers to transport any kind of drugs and exhibit advantageous properties based on an antimicrobial, anti-metastatic and/or anticancer activity of Ohmline itself and its capacity to transport any kind of drugs. This unique property is related to the unique capacity of Ohmline to adopt different lipid structures.

Hence, the present invention provides:
A liposome comprising a core and at least one lipid bilayer which comprises a glycerolipid of formula (I): 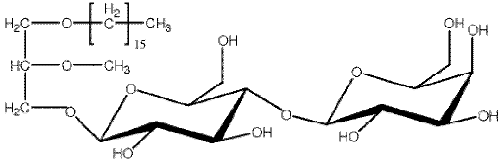
wherein the core comprises a hydrophilic anticancer agent or antimicrobial agent or any other hydrophilic drug, and/or
the lipid bilayer comprises a hydrophobic anticancer agent or antimicrobial agent or any other hydrophobic drug.

In a preferred embodiment, the core comprises a hydrophilic anticancer agent and/or the lipid bilayer comprises a hydrophobic anticancer agent.

In another embodiment, compatible with the previous one, the lipid bilayer lacks phospholipids or any other amphipathic lipid with a polar head and a double-chained apolar tail. In this embodiment, a hydrophobic anticancer agent, or antimicrobial agent or any other hydrophobic drug can be comprised in the lipid bilayer.

In another embodiment, the lipid bilayer consists of molecules of the glycerolipid of formula (I). This is compatible with the following embodiment, because the core can comprise a hydrophilic anticancer agent, or an antimicrobial agent or any other hydrophilic drug.

In other embodiment of the invention, the hydrophilic anticancer agent, the antimicrobial agent or the other hydrophilic drug is encapsulated within the core.

Preferably, the liposome has a diameter from 0.01 µm to 20 µm, preferably from 0.05 to 5 µm, more preferable from 2.5 to 5 µm, measured by microscope.

In other preferred embodiment of the invention, the hydrophilic or hydrophobic drug comprised in the liposome is an anticancer agent, and the anticancer agent is selected from the list consisting of; aromatase inhibitors, anti-estrogen, anti-androgen, a gonadorelin agonists, topoisomerase 1 inhibitors, topoisomerase 2 inhibitors, microtubule active agents, alkylating agents, anthracyclines, corticosteroids, IMiDs, protease inhibitors, IGF-1 inhibitors, CD40 antibodies, Smac mimetics, FGF3 modulators, mTOR inhibitors, HDAC inhibitors, IKK inhibitors, P38MAPK inhibitors, HSP90 inhibitors, akt inhibitors, antineoplastic agents, antimetabolites, platinum containing compounds, lipid- or protein kinase-targeting agents, protein- or lipid phosphatase-targeting agents, anti-angiogenic agents, agents that induce cell differentiation, bradykinin 1 receptor antagonists, angiotensin II antagonists, cyclooxygenase inhibitors, heparanase inhibitors, lymphokine inhibitors, cytokine inhibitors, bisphosphanates, anti-apoptotic pathway inhibitors, apoptotic pathway agonists, PPAR agonists, inhibitors of Ras isoforms, telomerase inhibitors, protease inhibitors, metalloproteinase inhibitors and aminopeptidase inhibitors, cytostatic agent, cytotoxic agent, taxane, antitumor antibiotic, topoisomerase II inhibitor, topoisomerase I inhibitor, tubulin interacting agent, antibodies, antiangiogenics, COX-2 inhibitors, hormonal agent, thymidilate synthase inhibitor, anti-metabolite, alkylating agent, farnesyl protein transferase inhibitor, signal transduction inhibitor, EGFR kinase inhibitor, antibody to EGFR, C-abl kinase inhibitor, hormonal therapy combination, aromatase inhibitor and combinations thereof.

In a more preferred embodiment of the invention the anticancer agent is a taxane selected from the list consisting of docetaxel, paclitaxel, tesetaxel, cabazitaxel; preferably, the taxane is docetaxel.

The invention also provides a gel of a glycerolipid of formula (I): 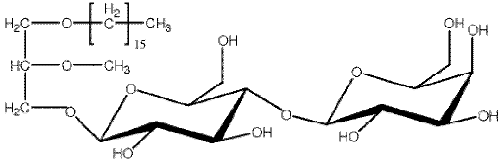 wherein the glycerolipid molecules form lipid nanotubes.

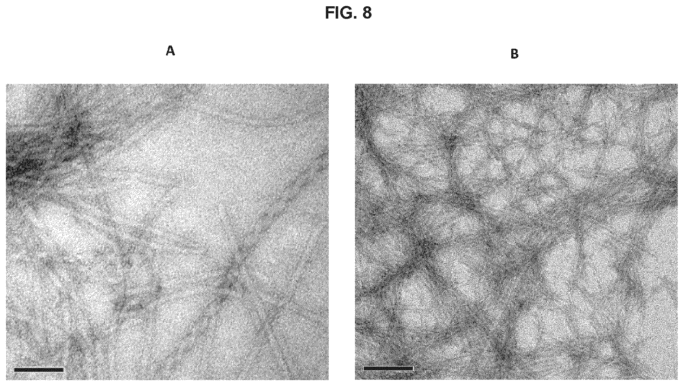

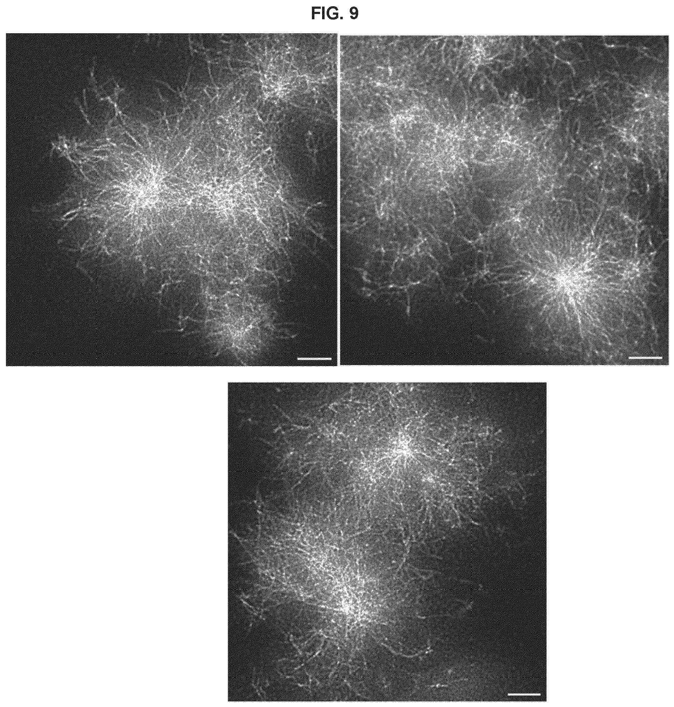

Preferably, the gel is a hydrogel (FIG. 8 and 9).

The liposome or the gel are useful as a DNA, siRNA, RNA and antibodies or nanobodies carriers.

The liposome or the gel are useful as a medicament for instance for the treatment and/or prophylaxis of a disease.

Thus, another aspect of the invention relates to the liposome or the gels, preferably hydrogels, of the previous aspects for use as medicaments.

Said medicament can be used as an antimicrobial medicament (for instance, an antibacterial medicament, an antiviral medicament), particularly if the liposome or the gel comprises and antimicrobial agent (additional to the glycerolipid of Formula (I), Ohmline), and can be for use in the treatment of a bacterial, viral, protozoan or fungal infection, particularly a biofilm-associated bacterial infection, either caused by Gram(+) or Gram(-) bacteria.

The liposomes or gels, or the pharmaceutical composition comprising any of them, can be also for use in the treatment of cancer and/or in the prevention or reduction of cancer metastasis. For instance, the use can be for primary growth inhibition or, as commented above, in the prevention or reduction of metastasis. When the liposomes or gels or the pharmaceutical compositions comprising them are for use in the treatment of cancer (either for primary growth inhibition or prevention or reduction of metastasis), preferably the liposomes or gels comprise an anticancer agent, additional to Ohmline itself. The cancer can be breast cancer, head and neck cancer, stomach cancer, prostate cancer, non-small-cell lung cancer, leukaemia, skin cancer, melanoma, liver cancer, ovarian cancer, pancreatic cancer, lung, kidney cancer, colon cancer, brain cancer, selected from gliomas and glioblastoma, thereof.

The invention also provides a pharmaceutical composition useful as a medicament comprising the liposome or the gel and at least one pharmaceutically acceptable excipient or/and one adjuvant. The pharmaceutically acceptable excipient and /or an adjuvant can be selected from the list consisting of diluents, buffers, preservatives, stabilisers, surfactants, solvents, disintegrants, antioxidants, binders, lubricants and colorants among other.

The pharmaceutical compositions of the present invention can be in the form of an oral, a topical, or an injectable, composition.

Said excipient can be selected from any of those known to a person skilled in the art and according to the route of administration to be used, the one expected for the therapeutic objective sought.

Oral compositions may be, for example, tablets, troches, lozenges, aqueous or oily suspensions, dispersible powders or granules, emulsions, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavouring agents, coloring agents and preserving agents in order to provide pharmaceutically elegant and palatable preparations.

Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc.

The tablets may be uncoated, or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate.

The aqueous suspensions may also contain one or more preservatives, for example ethyl, or n-propyl, p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or acetyl alcohol. Sweetening agents such as those set forth above, and flavouring agents may be added to provide a palatable oral preparation.

These compositions may be preserved by the addition of an antioxidant such as ascorbic acid. Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present. Syrups and elixirs may be formulated with sweetening agents, for example glycerol, propylene glycol, sorbitol or sucrose. Such formulations may also contain a demulcent, a preservative and flavouring and colouring agents

For injectable administration, the vehicle is usually made up of water and auxiliary substances required to make it compatible with physiological conditions, such as pH-regulating agents, tonicity agents, etc.

For topical administration, the excipients may be selected according to the pharmaceutical form intended in each case, preferably liquid or semisolid. Liquid formulations include aqueous solutions, hydroalcoholic solutions, lotions, etc. Semi-solid formulations can be ointments, creams or gels. Any of these formulations may require the use of preservatives, antioxidant agents, chelating agents, emulsifiers, pH regulating systems, tonicity regulating substances, preservatives, bioadhesive polymers, gelling substances, etc.

The preserving agent can be selected from, for example, sodium benzoate, ascorbic acid, parabens, bronopol, methylisothiazolinone, and mixtures thereof. The antioxidant can be chosen, for example, from butylated hydroxytoluene (BHT), hydroxyanisole and tocopherol and its derivatives. The chelating agent may be selected, for example, from the group consisting of ethylenediaminetetraacetic acid (EDTA), ethylene glycol bis-(2-aminoethyl)-tetraacetic acid (EGTA), and citric acid or its salts. The pH regulating system can be chosen, for example, between a phosphate buffer solution and a citrate buffer solution. The gelling agent can be selected, for example, from Carbopols, Poloxamers, Carboxymethylcellulose (CMC), Hydroxyethylcellulose (HEC), Hydroxypropyl-methyl (HPMC), Methylcellulose (MC), and mixtures thereof

In an embodiment of the invention, the stabilizer or the surfactant is an anionic/cationic lipid and/or it is present in a weight concentration in the composition from 5-45 % w/w.

Preferably, the pharmaceutical composition according to the present invention may be formulated as a vaccine carrier, for example, as RNA or DNA vaccines. Preferably, the stabilizer or the surfactant for RNA or DNA vaccines is a cationic lipid.

The pharmaceutical compositions according to the present invention are useful for the treatment or prevention of cancer, particularly, colorectal cancer, lung cancer, pancreas cancer, and gastric cancer or a composition for treating a cancer selected from colorectal cancer, lung cancer, gastric cancer, breast cancer, head and neck cancer, prostate cancer, non-small-cell lung cancer, leukaemia, skin cancer, liver cancer, ovarian cancer, pancreatic cancer, lung cancer and kidney cancer.

The pharmaceutical composition useful as a medicament comprising the liposome or the gel of the present invention may be used for preventing or reducing the occurrence of metastasis in any body tissue or in any body organ. The usefulness of a glycerolipid of formula (I) encompasses preventive or therapeutic treatments against the occurrence of metastasis in variety of tissues and organs including ovary, uterus, kidney, liver, lung, bone tissue (e.g. leg bones including femur, arm bones, spinal column including dorso-lumbar vertebra, pelvis), spleen, lymph nodes, colon, breast, brain, prostate, pancreas and skin.

The invention could be used in combination with other anticancer treatments, such as chemotherapy, cryotherapy, hyperthermia, radiation therapy, and the like.

This invention also encompasses a process for the preparation of a liposome, comprising at least the steps of:
i) providing a mixture which comprises
   an aqueous buffer;
   at least one anticancer or antimicrobial agent or any other drug; and
   the glycerolipid of formula (I); 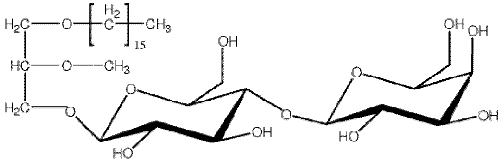
   wherein the concentration of the glycerolipid of formula (I) in the mixture is equal or lower than 3 mg/ml; preferably from 0,04 mg/ml up to 3 mg/ml;
ii) stirring the mixture at a temperature higher than 40 ºC, preferably, from 40 °C to 80 °C
   wherein when the anticancer or antimicrobial agent or the other added drug is a hydrophobic compound the anticancer or antimicrobial agent or the other added drug is added to the mixture of step i) with the glycerolipid of formula (I) and
   wherein when the anticancer or antimicrobial agent or the other added drug is a hydrophilic compound the anticancer agent is incorporated in the aqueous buffer.

In a preferable embodiment of the invention, the hydrophobic compound is dissolved in polar solvent to be dried together with Ohmline. Both compounds: the hydrophobic compound (anticancer or antimicrobial agent or other drug) to be co-encapsulated with Ohmline as well as Ohmline are co-dissolved together in a polar buffer to be dried with a stream of hydrogen in order to forms a film which is then hydrated together with the aqueous buffer.

In a preferable embodiment of the invention, the glycerolipid of formula (I) is a powder which is firstly dissolved in a solvent or mixture of solvents such as chloroform/methanol (preferably 3:1), then dried, preferably under vacuum and/or under Nitrogen atmosphere, to form a film which is rehydrated in a buffer such as Phosphate-buffered saline to pH=7,6.

The process for the preparation of a liposome further comprises the step (iii), of treating the mixture obtained in step (ii) by extrusion, preferably by means of a membrane, or sonication.

The invention also encompasses a process for the preparation of a gel comprising at least the steps of:
i) providing a mixture which comprises:
   an aqueous buffer;
   at least one anticancer or antimicrobial agent or any other drug; and
   the glycerolipid of formula (I) 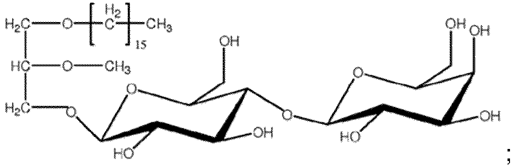
   wherein the concentration of the glycerolipid of formula (I) in the mixture is higher than 3 mg/ml, more preferably, concentration of the glycerolipid of formula (I) in the mixture is 3 to 25 mg/l;
ii) stirring the mixture at a temperature higher than 40 ºC, preferably between 40 to 80° C
   wherein when the anticancer or antimicrobial agent or the other added drug is a hydrophobic compound the anticancer or antimicrobial agent or the other drug is added to the mixture with the glycerolipid of formula (I) and
   wherein when the anticancer or antimicrobial agent or the other added compound is a hydrophilic compound the anticancer agent is incorporated in the aqueous buffer.

Preferably, the aqueous buffer is selected from the list consisting of Phosphate Buffer Saline (PBS), NaCl buffer, sterilized water, and combinations thereof.

According to the present invention, data show that, Ohmline form lipid vesicles that have the capacity to work as drug transporter. Interestingly these Ohmline vesicles alone (empty) acts as a new anti-tumor agent, probably due to the enhanced permeability and retention (EPR) effect, by which they tend to accumulate in tumor tissue much more than in normal tissues.

This novel property of Ohmline makes clear that this lipid can not only prevent metastases but also can behave as an anti-tumor agent by itself and moreover carry and deliver another antitumor agent showing a synergetic effect.

Thus, these multiple properties have never been demonstrated so far for a pharmacologic carrier. Cancer and metastasis prevention, together with the ability to deliver drugs is a major inventive breakthrough in the technical field of the invention and an improvement in the efficacy of chemotherapy.

### EXAMPLES

In the following Section it is described the preparation of a new transporter that combines multiple pharmacological activities. To reach this objective the present inventors have designed and prepared a transporter (Ohmline) which is already pharmacologically active by itself. The structure of the transporter would allow the insertion of hydrophobic and hydrophilic drugs and would confer to the same carrier a large diversity of therapeutic properties.

In order to demonstrate the advantageous property of the transporter of the present invention, the inventors have performed the biophysical characterization of the Ohmline lipid phases and the encapsulation of hydrophilic probe (carboxifluorescein) and hydrophobic drugs like taxanes (docetaxel) as well as tests *in-vitro* and *in-vivo* thereof. In addition, the invention discloses test in-vivo with a formulation according to the present invention named as TAXOM for different type of cancers and docetaxel concentrations. (e.g. breast cancer).

### 1-General Vesicles preparation

Ohmline was synthesized as previously described (Girault A, et al. New alkyl-lipid blockers of SK3 channels reduce cancer cell migration and occurrence of metastasis. Current cancer drug targets. 2011;11:1111-1125).

Ohmline powder is dissolved in chloroform:methanol mixture (3:1), evaporated under nitrogen flow and desiccated overnight under vacuum to remove any residual solvent. Dried films are vortexed over 40°C in the buffer chosen for each essay.

The hydrated Ohmline sheets detach during agitation and form MLV (multilamellar vesicles); LUV (large unilamellar vesicles) are prepared by extrusion or sonication (SUV).

### 1.1-Visualization of MLV by Optical Microscopy

Ohmline multilamellar vesicles labelled with the hydrophobic probe BODIPY (Thermo Fisher Scientific product D3922, https://www.thermofisher.com/order/catalog/product/D3922) were prepared. The MLV result in the size range between 500 nm to 2,5 µm. The FIG. 2 shows Fluorescence microscopy Images of Ohmline MLV 1mg/ml labelled with BODIPY 493/503, in the size range between 500 nm to 2.5 µm.

### 1.2-Visualization of LUV by Electron Microscopy

With MLV of Ohmline at different concentration, LUV samples were prepared by sonication or extrusion. Formvar/carbon-coated nickel grids were deposited on a drop of samples during five minutes and rinsed two times on drop of water. The negative staining was then performed with three consecutive contrasting steps using 2% uranyl acetate (Agar Scientific, Stansted, UK), before analysis under the transmission electron microscope (JEOL 1011, Tokyo, Japan). Unilamellar lipid vesicles of 100 nm size were prepared by extrusion on mini-extruder Avanti Polar Lipids using polycarbonate membrane pore of 100 nm diameter. Unilamellar vesicles of about 50 nm were prepared in a sonication bath over 50°C.

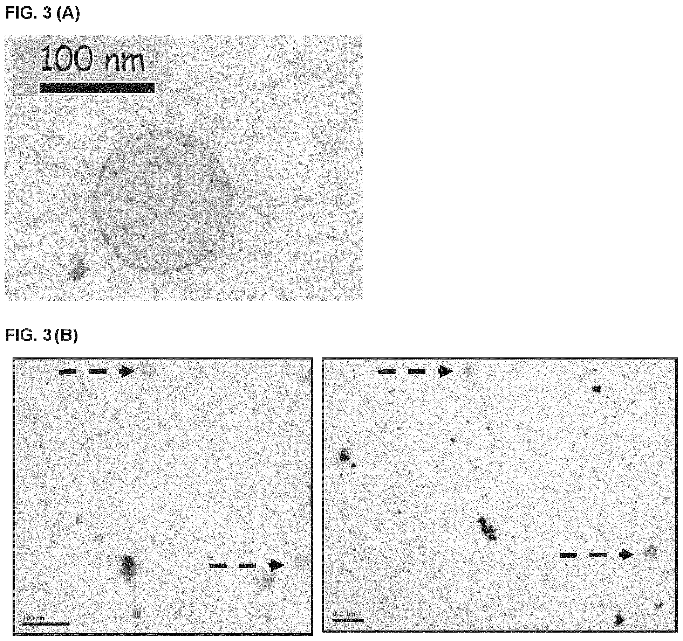

The FIG. 3 A and B show Ohmline final dilution on the grid of 100 µM, and 20 µM respectively.

### 2-Hydrophilic probes Carboxyfluorescein encapsulated into Ohmline multilamellar vesicles.

Dry film of Ohmline at 0.8 mg/ml (1,23 mM) is mixed with 400 µM Carboxyfluorescein (CF) (6-carboxyfluorescein, Sigma Aldrich product C0662: https://www.sigmaaldrich.com/ES/es/product/sigma/c0662) solution in PBS buffer, pH=7,6. Frequent freeze thawings and vortexings allow the entrapping of the probe inside the Ohmline structure. Separation of free and encapsulated CF on a PD-10 pre-packed column (Cytiva: https://www.cytivalifesciences.com/en/us/shop/chromatography/prepacked-columns/desalting-and-buffer-exchange/sephadex-g-25-in-pd-10-desalting-columns-p-05778), Sephadex G-25 M allows to visualize CF entrapment (green).

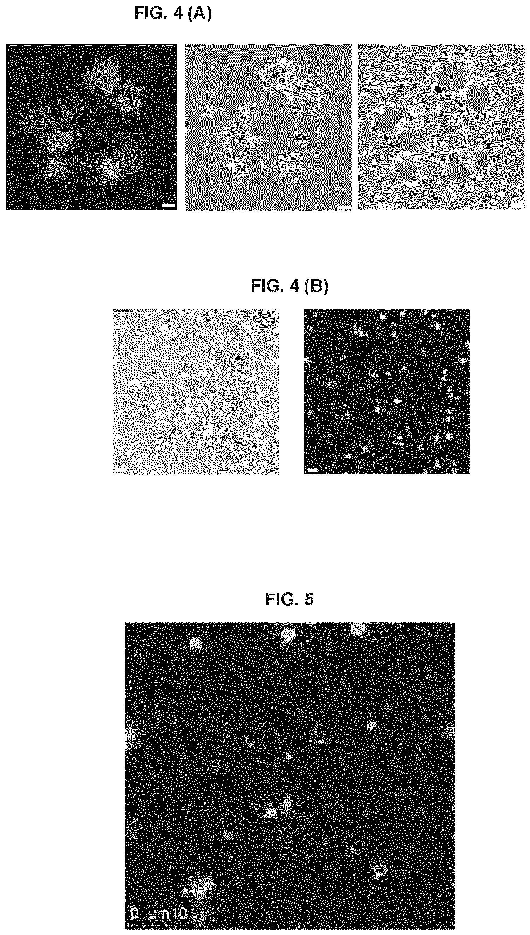

FIG 4 A and B show phase contrast and fluorescence microscopy images (λex=492nm, λem=517nm) of the Ohmline MLV with carboxyfluorescein encapsulated inside the lamellas.

The MLV vesicles of Ohmline loaded with CF show the regular size of a multilamellar particle between 2.5 and 5 µm.

### 3-Lipophilic probes

Any lipophilic (or hydrophobic) molecule will insert into the lipid bilayer of Ohmline vesicles as illustrated with di-4-ANNEPDHQ fluorescent probe (Thermo Fisher Scientific product D36802: https://www.thermofisher.com/order/catalog/product/D36802). Ohmline liposomes were incubated with 2 µl of DMSO stock solution of di-4-ANNEPDHQ at a Lipid/Probe ratio = 100. Final concentration: Ohmline 1.23 mM and di-4-ANNEPDHQ 12.3 µM.

FIG. 5 shows a Microscopy Images of Ohmline MLV labelled with di4ANNEPDHQ. Λ excit= 488 nm λ emiss= 520 to 700 nm. In this case, Ohmline vesicles with lipophilic probe inserted in the bilayer have a particles size between 1 to 5 µm.

### 4-Docetaxel encapsulated in Ohmline MLV

Docetaxel was purchased from Sigma-Aldrich (Sigma-Aldrich product 01885-5MG-F, https://www.sigmaaldrich.com/ES/es/search/01885-5mg-f?focus=products&page=1&perpage=30&sort=relevance&term=01885-5mg-f&type=product). It was used to prepare liposomes and also, as explained below, to prepare a composition similar to the commercial medicament TAXOTERE, containing 20 mg docetaxel per 0.5 ml TWEEN 80 as excipient and ethanol 95% (v/v) in H₂O 13/87 p/p).

Ohmline liposomes loaded with docetaxel are named herein TAXOM. The TAXOM concentration is usually referred as the ratio (Docetaxel nM/Ohmline µM) and the abbreviation TAX for TAXOM and DOX for docetaxel, showing the liposomes of Ohmline fuse with the cells and release their cargo.

The MLV Ohmline liposomes were prepared as described in section 1 above in PBS buffer, pH=7.6.

The liposomes were formed by hydration of the film to solubilize Ohmline with docetaxel intercalated in the bilayer in a ratio indicated in each experiment. The TAXOM particles where prepared fresh and used during the day.

### 4.1- In-vitro cell viability test

Ohmline nanoparticles were used to deliver docetaxel into different cancer cell lines. Cell viability was determined using tetrazolium salt reduction method (MTT) (CyQUANT^{™} MTT Cell Viability Assary, Thermo Fisher Scientific product V13154: https://www.thermofisher.com/order/catalog/product/V13154?ef_id=4431670897231bd9f4af0 436727d3335:G:s&s_kwcid=AL!3652!10!77240846955634!77240903742099&cid=bid_pca_i va _r01_co_cp1359_pjt0000_bid00000_0se_bng_nt_pur_con&msclkid=4431670897231bd9f 4af0436727d3335#/V13154), that reflects the number of viable cells. 6500 cells were seeded at day=0 in a 48 wells plate. Each well was filled up with the regular medium of the cancer cell. At day=1 the medium was aspirated and replaced with new media for each formulation to be tested. Cells were incubated up to 3 days.

At the end of the incubation, the medium was aspirated and replaced with MTT solution at 0,5 mg/ml concentration. The plate was incubated at 37ºC by 30-45 min. After incubation the supernatant was aspirated, and the formed crystals dissolved with DMSO and gently agitation at room temperature protected from the light. 200 µl were transferred from each well to 96 well plates to measure the absorbance at 570 nm. (see Figures 8A, 8B and 8C).

### Formulations

Ohmline control at 1 and 5 µM, in MLV formulation.

DOX is docetaxel at 0,5 and 1 nM, in DMSO.

TAX design MLV of Ohmline that were co-solubilize with docetaxel at different molar ratios in chloroform: methanol mixture (3:1):
**TAX 0,5/1** =0,0005 docetaxel/Ohmline, **TAX 0,5/5**=0,0001 docetaxel/Ohmline
**TAX 1/1** = 0,001 docetaxel/Ohmline, **TAX 1/5** = 0,0002 docetaxel/Ohmline

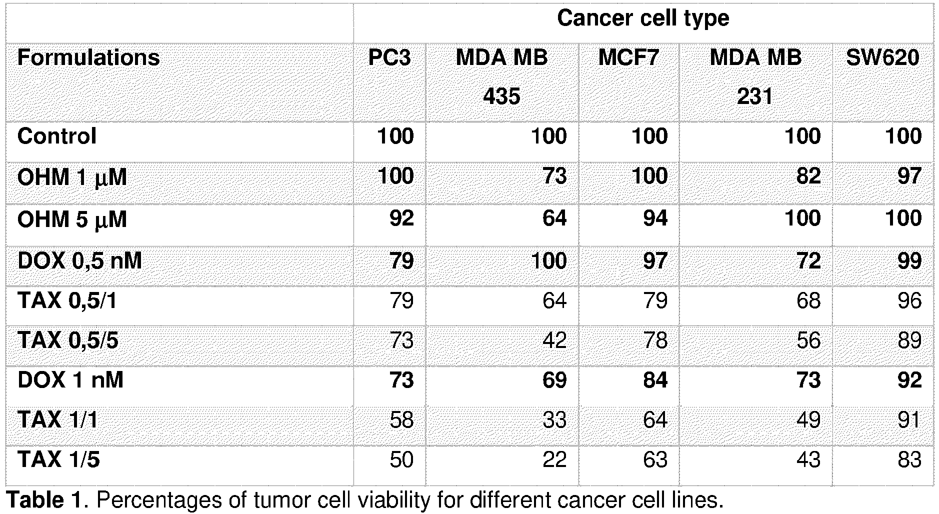

In some conditions we observed a decrease in the viability of the cells only after Ohmline empty vesicles treatment. The TAX formulation was always more efficient than DOX and the higher efficiency was observed at higher amounts of Ohmline in TAX (e.g. TAX=0,5/5 is better than TAX=0,5/1).

Empty Ohmline vesicles can work as an anti-tumor agent.

Ohmline vesicles loaded with docetaxel (TAX) are much more efficient than Ohmline or docetaxel treatment alone (synergic effect).

### 4.2 - In vivo animal model

The inventors have used 48 nude female NMRI mice (Janvier Laboratories, France: https://janvier-labs.com/), 4 weeks old, 12 mice per treatment. Model cancer: MDA-MB-435s mammary Fat Pad breast cancer. Mice were bred and housed at Inserm U892 (Nantes University) under the animal care license Nº 44565). Mice were injected (intravenously) 2 weeks after being grafted with cancer cells. Duration: 2 months for lung metastases detection.

### Treatment protocol:

| **DAY** | | | | | | | | | |
|---|---|---|---|---|---|---|---|---|---|
| **0** | **13** | **16** | **20** | **23** | **29** | **35** | **42** | **49** | **55** |
| 2x10⁶cell injection | Primary tumor grow | **1^{st} injection** | **2^{nd} injection** | **3^{rd} injection** | **4^{th} injection** | Tumor and metastasis development | Tumor and metastasis development | Tumor and metastasis development | Ex-vivo tumor and metastasis evaluation |

### Formulations

Group A/CONTROL: NaCl 150 mM via i.v. route - 2 injections per week - 2 weeks

Group B/Ohmline: Ohmline 15mg/Kg = 3 mg/ml of Ohmline in NaCl 150 mM for 30 gr. Mice i.v. route- 2 injections per week - 2 weeks.

Group C/Docetaxel formulated in the inventors' laboratory as a Taxotere composition: 5 mg/Kg i.v. - 2 injections per week - 2 weeks.

Group D/TAXOM: 15 mg/Kg (3mg/ml Ohmline in NaCl 154mM (idem group A) + 5 mg/Kg docetaxel (stock of 100mg/ml DMSO - 1,5 µl) i.v.route - 2 injections per week - 2 weeks.

MLV of Ohmline were co-solubilized with docetaxel in chloroform: methanol mixture to prepare MLV with both components together. Molar ratio : 0.4 docetaxel/Ohmline.

**Table 2 Ex-vivo analysis. Normalize average reduction in the tumor size for each formulation essayed.**

| **Treatment** | **Normalize average reduction** |
|---|---|
| **Control** | 0 |
| **Docetaxel** - **Taxotere** | 38 |
| **Ohmline MLV** | 39,5 |
| **TAX** | 44,5 |

**Table 3. Ex-vivo analysis. Percentage of metastasis development for each formulation essayed.**

| **Treatment** | **Organs metastasis development** | | |
|---|---|---|---|
| | Ganglion | uterus/ovary | lung |
| **Control** | 50 | 25 | 83 |
| **Docetaxel** - **Taxotere** | 11 | 24 | 78 |
| **Ohmline MLV** | 33 | 8 | 50 |
| **TAX** | 17 | 8 | 58 |

### Conclusions

The main contribution to the prior art is that Ohmline vesicles alone are as efficient as the other drug formulation in reducing the tumor size.

Anti-metastatic activity was optimal with TAX, meaning that combination of Ohmline with docetaxel (even when the ratio than we have chosen was not the best) improves the quality of the treatment.

A very important point to mention is that 2 mice died after the treatment with taxotere, and none with Ohmline MLV or its combination with docetaxel.

### 5. General hydrogel preparation

Ohmline powder is dissolved in chloroform:methanol mixture (3:1), evaporated under nitrogen flow and desiccated overnight under vacuum to remove any residual solvent. Dried films are vortexed over 40°C in the buffer chosen for each essay. The hydrated Ohmline in a concentration higher than 3 mg/ml turns viscous.

### Macroscopic appearance: Viscous hydrogel.

4 mg of Ohmline (6,10 mM) where dissolved in organic solvent (chloroform:methanol mixture 3:1) in the presence of tetramethylindocarbocyanine (61µM) (Sigma-Aldrich, product 42364: https://www.sigmaaldrich.com/ES/es/product/sigma/42364) to obtain 100/1 molar ratio for Ohmline/probe. The solvent was evaporated under nitrogen flow and desiccated overnight. The film was hydrated in miliQ water and vortexed over 40°C. The appearance of Ohmline gel was observed in vitro as well as the ability to encapsulated the lipophilic probe: tetramethylcarbocyanine.

### Formability and degradation in vitro

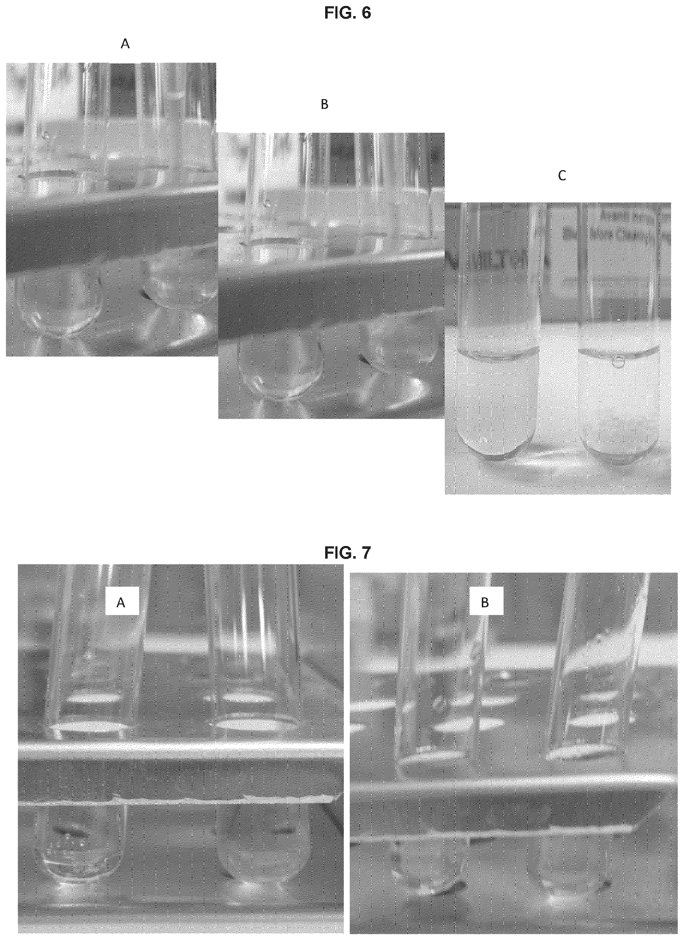

2 transparent tubes with 1 ml of milliQ water were used to visualize the Ohmline hydrogel behavior. For the control, tetramethylindocarbocyanine was dissolved in methanol. The same final concentration of probe was dissolved in each tube. The FIG 6 photographs show the sequence in time for A, B and C. In all the figures the left tube contains the methanol solution probe that quickly spread in the medium. However, the Ohmline gel that was injected into water maintain his phase for some days. The accumulation of the probe inside the gel can be easily observed (Fig 6 C). After 2 days the gel remains on the bottom Fig 7 A, decreasing gradually. At day 3 the gel was completely cleared (become vesicles that keep the probe in solution) FIG 7 B.

It can be concluded that the hydrogel, which forms a sustained-release depot in vitro, may serve as a promising delivery system for cancer in situ treatment or even for infection treatment.

### Microscopic appearance: Nanotubes that create the viscous network.

The Ohmline hydrogel was prepared at 10 mg/ml and the sample was visualized by Electron Microscopy following the same protocol that was previously described for LUVs. The FIG 8 shows an extensive network of nanotubes that intersect in all positions.

### Microscopic appearance: Nanotubes as a carrier of lipophilic probe

The same Ohmline hydrogel of 4mg/ml with tetramethylcarbocyanine previously characterized was used here to observe the nanotubes and the ability to carry the probe. An STED Microscope was used to improve resolution. The FIG. 9 shows the same extensive network of nanotubes observed by Electron Microscopy but this time with the lipophilic probe incorporated in the lipid structure. Ohmline gel can incorporate hydrophobic drug and used to in-situ drug delivery avoiding the side effect of systemic drug circulation.

### 5- Antimicrobial activity

The reference strains *Staphylococcus aureus* ATCC 25923 and *Escherichia coli* ATCC 29923 (ATCC: American Type Culture Collection) were employed, as examples of Gram positive (Gram(+)) or Gram negative (Gram(-)) bacteria, respectively. For bacteria culture the Mueller Hinton Broth medium from Britania Lab S.A. (www.britanialab.com) was used.

All experiments were carried out by making a depot of 20 µl of 5mg/ml Ohmline gel in a 96-well plate where 200 µl of bacteria were cultured starting from an initial OD at 600 nm of 0.15. The OD 600 was recorded at several time intervals during 24 h. Cultures were incubated at 37°C with agitation. All the experiments were carried out by treating bacteria with Ohmline hydrogel depot (100% Ohmline composition), resulting on the release of Ohmline into the media (final concentration 0.5mg/mL).

As can be seen in FIG.10, Ohmline hydrogel depot exhibited direct antibacterial activity against *E. coli and S. aureus* with a MIC (minimal inhibitory concentration) for *S*. *aureus* of 0.5 µg/ml.

The microscopy images (FIG: 10 B) show an impairment at the level of the plasma membrane in both species that emphasizes the role of this nanomaterial as potentiator to increase the activity of other antibacterial agents and/or reversing antimicrobial resistance.

More importantly, some preclinical data in animal models confirm the absence of any toxicity using i.v. and oral administration of Ohmline nanomaterial up to concentrations of 500 mg/Kg in rats. ADME (absorption, distribution, metabolism and excretion) studies also reveal broad distribution and tropism to tissues and organs. Ohmline also showed distribution to brain (able to cross the blood brain barrier).

## Claims

1. A liposome comprising
a core and
at least one lipid bilayer which comprises a glycerolipid of formula (I): 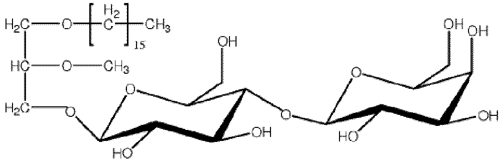
wherein the core comprises a hydrophilic anticancer agent or antimicrobial agent or any other hydrophilic drug and/or
the lipid bilayer comprises a hydrophobic anticancer agent or antimicrobial agent or any other hydrophobic drug.

2. The liposome according to claim 1, wherein the lipid bilayer is composed of the glycerolipid of formula (I):

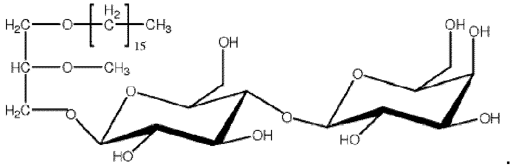

3. The liposome according to any one of claims 1-2, wherein a hydrophilic anticancer agent is encapsulated within the core.

4. The liposome according to any of the preceding claims, wherein the liposome comprises an anticancer agent and the anticancer agent is selected from the list consisting of: aromatase inhibitors, anti-estrogen, anti-androgen, a gonadorelin agonists, topoisomerase 1 inhibitors, topoisomerase 2 inhibitors, microtubule active agents, alkylating agents, anthracyclines, corticosteroids, IMiDs, protease inhibitors, IGF-1 inhibitors, CD40 antibodies, Smac mimetics, FGF3 modulators, mTOR inhibitors, HDAC inhibitors, IKK inhibitors, P38MAPK inhibitors, HSP90 inhibitors, akt inhibitors, antineoplastic agents, antimetabolites, platinium containing compounds, lipid- or protein kinase-targeting agents, protein- or lipid phosphatase-targeting agents, anti-angiogentic agents, agents that induce cell differentiation, bradykinin 1 receptor antagonists, angiotensin II antagonists, cyclooxygenase inhibitors, heparanase inhibitors, lymphokine inhibitors, cytokine inhibitors, bisphosphanates, anti-apoptotic pathway inhibitors, apoptotic pathway agonists, PPAR agonists, inhibitors of Ras isoforms, telomerase inhibitors, protease inhibitors, metalloproteinase inhibitors and aminopeptidase inhibitors, cytostatic agent, cytotoxic agent, taxane, antitumor antibiotic, topoisomerase II inhibitor, topoisomerase I inhibitor, tubulin interacting agent, antibodies, antiangiogenics, COX-2 inhibitors, hormonal agent, thymidilate synthase inhibitor, anti-metabolite, alkylating agent, farnesyl protein transferase inhibitor, signal transduction inhibitor, EGFR kinase inhibitor, antibody to EGFR, C-abl kinase inhibitor, hormonal therapy combination, aromatase inhibitor and combinations thereof.

5. The liposome according to the previous claim, wherein the anticancer agent is a taxane selected from the list consisting of docetaxel, paclitaxel, tesetaxel, cabazitaxel.

6. A gel of a glycerolipid of formula (I): 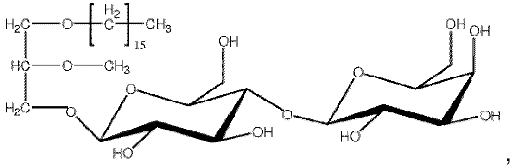 wherein the glycerolipid molecules form lipid nanotubes.

7. The liposome or the gel according to the previous claims for use as a DNA, siRNA, RNA, antibodies or nanobodies carrier.

8. A pharmaceutical composition comprising a liposome according to any of the claims 1-5 or comprising the gel according to claim 6, and at least one pharmaceutically acceptable excipient or/and one adjuvant.

9. The pharmaceutical composition according to the previous claim wherein the one pharmaceutically acceptable excipient is selected from the list consisting of a stabiliser or a surfactant, wherein the stabilizer or the surfactant is an anionic/cationic lipid and/or the stabilizer and the surfactant are in a concentration in the composition from 5-45 % w/w, to the total weight of the composition.

10. A pharmaceutical composition according to any one of the claims 8-9, wherein the composition is a vaccine.

11. The liposome according to any one of claims 1-5 or the gel according to claim 6 or the pharmaceutical composition according or any one of the claims 8-9 for use as a medicament.

12. The liposome or the gel for use according to claim 11, wherein the liposome or the gel comprises an antimicrobial agent and which is for use in the treatment of a bacterial, a viral, a protozoan or a fungal infection.

13. The liposome or the gel for use according to claim 12, wherein the infection is a biofilm-associated bacterial infection.

14. The liposome or the gel for use according to any one of claims 12 or 13, wherein the infection is a bacterial infection caused by *Escherichia coli* or *Staphylococcus aureus.*

15. The liposome or the gel or the pharmaceutical composition for use according to claim 11, for use in the treatment or prevention of cancer and/or in the prevention or reduction of cancer metastasis, wherein the liposome or gel comprise an anticancer agent.

16. The liposome or the gel or the pharmaceutical composition for use according to claim 15, wherein the use in the treatment of cancer is for primary tumor growth inhibition or reduction.

17. The liposome or the gel or the pharmaceutical composition for use according to claim 15, wherein the cancer is selected form the list consisting of: breast cancer, head and neck cancer, stomach cancer, prostate cancer, non-small-cell lung cancer, leukaemia, skin cancer, melanoma, liver cancer, ovarian cancer, pancreatic cancer, lung cancer, kidney cancer, colon cancer, brain cancer, selected from gliomas and glioblastoma, thereof.

18. A process for the preparation of a liposome as defined in any of the claims 1-5, comprising at least the steps of:
(i) providing a mixture which comprises
an aqueous buffer;
at least one anticancer agent or one antimicrobial agent or any other drug; and
the glycerolipid of formula (I);
wherein the concentration of the glycerolipid of formula (I) in the mixture is equal or lower than 3 mg/ml;
ii) stirring the mixture at a temperature higher than 40 ºC
wherein, when the anticancer or antimicrobial agent or the other drug is a hydrophobic compound, the anticancer or antimicrobial agent or the other drug is added to the mixture with the glycerolipid of formula (I) obtained in the previous step and
wherein, when the anticancer or antimicrobial agent or the other drug is a hydrophilic compound, the anticancer agent is incorporated in the aqueous buffer.

19. The process according to claim 18, wherein the hydrophobic anticancer or antimicrobial agent or drug of step i) was previously dried before mixed with the buffer.

20. The process according to any of the claims 18-19, which further comprises the step (iii), which comprises treating the mixture obtained in step (ii) by extrusion, or sonication.

21. A process for the preparation of the gel as defined in claim 6, comprising at least the steps of:
(i) providing a mixture which comprises
an aqueous buffer;
at least one anticancer agent or one antimicrobial agent or any other drug; and
the glycerolipid of formula (I);
wherein the concentration of the glycerolipid of formula (I) in the mixture is higher than 3 mg/ml;
ii) stirring the mixture at a temperature higher than 40 ºC,
wherein when the anticancer or antimicrobial agent or the other drug is a hydrophobic compound, the anticancer or antimicrobial agent or the other drug is added to the mixture with the glycerolipid of formula (I) and
wherein when the anticancer or antimicrobial agent or the other drug is a hydrophilic compound, the anticancer or antimicrobial agent or the other drug is incorporated in the aqueous buffer.

## Patentansprüche

1. Liposom, umfassend:
einen Kern und
mindestens eine Lipiddoppelschicht, die ein Glycerolipid der Formel (I) umfasst: 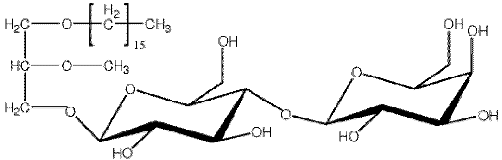
wobei der Kern einen hydrophilen Antikrebswirkstoff oder antimikrobiellen Wirkstoff oder jedes andere hydrophile Arzneimittel umfasst und/oder
die Lipiddoppelschicht einen hydrophoben Antikrebswirkstoff oder antimikrobiellen Wirkstoff oder jedes andere hydrophobe Arzneimittel umfasst.

2. Liposom nach Anspruch 1, wobei die Lipiddoppelschicht aus dem Glycerolipid der Formel (I) besteht:

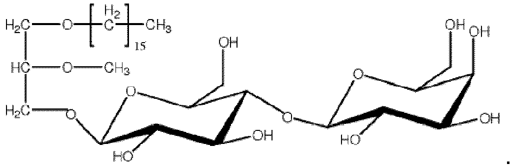

3. Liposom nach einem der Ansprüche 1-2, wobei ein hydrophiler Antikrebswirkstoff in den Kern eingekapselt ist.

4. Liposom nach einem der vorhergehenden Ansprüche, wobei das Liposom einen Antikrebswirkstoff umfasst und der Antikrebswirkstoff ausgewählt ist aus der Liste bestehend aus: Aromataseinhibitoren, Anti-Östrogenen, Anti-Androgenen, Gonadorelin-Agonisten, Topoisomerase-1-Inhibitoren, Topoisomerase-2-Inhibitoren, Mikrotubuli-aktiven Wirkstoffen, Alkylanzien, Anthracyclinen, Kortikosteroiden, IMiDs, Proteaseinhibitoren, IGF-1-Inhibitoren, CD40-Antikörpern, Smac-Mimetika, FGF3-Modulatoren, mTOR-Inhibitoren, HDAC-Inhibitoren, IKK-Inhibitoren, P38MAPK-Inhibitoren, HSP90-Inhibitoren, Akt-Inhibitoren, antineoplastischen Wirkstoffen, Antimetaboliten, platinhaltigen Verbindungen, auf Lipid- oder Proteinkinasen abzielenden Wirkstoffen, auf Protein- oder Lipidphosphatasen abzielenden Wirkstoffen, antiangiogentischen Wirkstoffen, Wirkstoffen, die die Zelldifferenzierung induzieren, Bradykinin-1-Rezeptor-Antagonisten, Angiotensin-II-Antagonisten, Cyclooxygenase-Inhibitoren, Heparanase-Inhibitoren, Lymphokin-Inhibitoren, Zytokin-Inhibitoren, Bisphosphanaten, Inhibitoren des anti-apoptotischen Weges, Agonisten des apoptotischen Weges, PPAR-Agonisten, Inhibitoren der Ras-Isoformen, Telomerase-Inhibitoren, Protease-Inhibitoren, Metalloproteinase-Inhibitoren und Aminopeptidase-Inhibitoren, Zytostatika, zytotoxischen Wirkstoffen, Taxanen, Antitumor-Antibiotika, Topoisomerase-II-Inhibitoren, Topoisomerase-I-Inhibitoren, Tubulininteragierenden Wirkstoffen, Antikörpern, Antiangiogenika, COX-2-Hemmern, hormonellen Wirkstoffen, Thymidilat-Synthase-Hemmern, Anti-Metaboliten, Alkylanzien, Farnesyltransferase-Inhibitoren, Signaltransduktionshemmern, EGFR-Kinase-Inhibitoren, Antikörpern gegen EGFR, C-abl-Kinase-Inhibitoren, Hormontherapie-Kombinationen, Aromatasehemmern und Kombinationen davon.

5. Liposom nach dem vorhergehenden Anspruch, wobei der Antikrebswirkstoff ein Taxan ist, ausgewählt aus der Liste bestehend aus Docetaxel, Paclitaxel, Tesetaxel, Cabazitaxel.

6. Gel aus einem Glycerolipid der Formel (I): 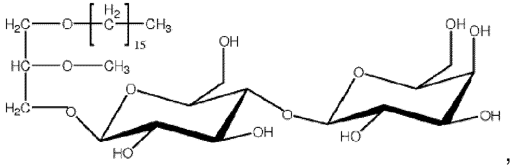 wobei die Glycerolipidmoleküle Lipid-Nanoröhrchen bilden.

7. Liposom oder Gel nach den vorhergehenden Ansprüchen zur Verwendung als DNA-, siRNA-, RNA-, Antikörperoder Nanokörperträger.

8. Pharmazeutische Zusammensetzung, die ein Liposom nach einem der Ansprüche 1-5 oder das Gel nach Anspruch 6 und mindestens einen pharmazeutisch verträglichen Hilfsstoff oder/und ein Adjuvans umfasst.

9. Pharmazeutische Zusammensetzung nach dem vorhergehenden Anspruch, wobei der eine pharmazeutisch verträgliche Hilfsstoff aus der Liste ausgewählt ist, die aus einem Stabilisator oder einem oberflächenaktiven Mittel besteht, wobei der Stabilisator oder das oberflächenaktive Mittel ein anionisches/kationisches Lipid ist und/oder der Stabilisator und das oberflächenaktive Mittel in der Zusammensetzung in einer Konzentration von 5-45 Gew.-%, bezogen auf das Gesamtgewicht der Zusammensetzung, vorliegen.

10. Pharmazeutische Zusammensetzung nach einem der Ansprüche 8-9, wobei die Zusammensetzung ein Impfstoff ist.

11. Liposom nach einem der Ansprüche 1-5 oder Gel nach Anspruch 6 oder die pharmazeutische Zusammensetzung nach einem der Ansprüche 8-9 zur Verwendung als Medikament.

12. Liposom oder Gel zur Verwendung nach Anspruch 11, wobei das Liposom oder Gel einen antimikrobiellen Wirkstoff umfasst, der zur Behandlung einer bakteriellen, viralen, protozoischen oder Pilzinfektion verwendet wird.

13. Liposom oder Gel zur Verwendung nach Anspruch 12, wobei die Infektion eine Biofilm-assoziierte bakterielle Infektion ist.

14. Liposom oder Gel zur Verwendung nach einem der Ansprüche 12 oder 13, wobei die Infektion eine bakterielle Infektion ist, die durch *Escherichia coli* oder *Staphylococcus aureus* verursacht wird.

15. Liposom oder Gel oder pharmazeutische Zusammensetzung zur Verwendung nach Anspruch 11 zur Verwendung bei der Behandlung oder Vorbeugung von Krebs und/oder bei der Vorbeugung oder Verringerung von Krebsmetastasen, wobei das Liposom oder das Gel einen Antikrebswirkstoff umfassen.

16. Liposom oder Gel oder pharmazeutische Zusammensetzung zur Verwendung nach Anspruch 15, wobei die Verwendung bei der Behandlung von Krebs zur primären Tumorwachstumshemmung oder -verringerung erfolgt.

17. Liposom oder Gel oder pharmazeutische Zusammensetzung zur Verwendung nach Anspruch 15, wobei der Krebs ausgewählt ist aus der Liste bestehend aus: Brustkrebs, Kopf- und Halskrebs, Magenkrebs, Prostatakrebs, nichtkleinzelligem Lungenkrebs, Leukämie, Hautkrebs, Melanom, Leberkrebs, Eierstockkrebs, Bauchspeicheldrüsenkrebs, Lungenkrebs, Nierenkrebs, Dickdarmkrebs, Gehirnkrebs, der ausgewählt ist aus Gliomen und Glioblastom.

18. Verfahren zur Herstellung eines Liposoms, wie in einem der Ansprüche 1-5 definiert, das mindestens die folgenden Schritte umfasst:
(i) Bereitstellung eines Gemischs, das Folgendes umfasst:
einen wässrigen Puffer;
mindestens einen Antikrebswirkstoff oder einen antimikrobiellen Wirkstoff oder jedes andere Arzneimittel; und das Glycerolipid der Formel (I);
wobei die Konzentration des Glycerolipids der Formel (I) in dem Gemisch gleich oder niedriger als 3 mg/ml ist;
ii) Rühren des Gemischs bei einer Temperatur von mehr als 40 ºC
wobei, wenn der Antikrebswirkstoff oder der antimikrobielle Wirkstoff oder das andere Arzneimittel eine hydrophobe Verbindung ist, der Antikrebswirkstoff oder der antimikrobielle Wirkstoff oder das andere Arzneimittel dem Gemisch mit dem im vorhergehenden Schritt erhaltenen Glycerolipid der Formel (I) hinzugefügt wird, und wobei, wenn der Antikrebswirkstoff oder der antimikrobielle Wirkstoff oder das andere Arzneimittel eine hydrophile Verbindung ist, der Antikrebswirkstoff in den wässrigen Puffer eingearbeitet wird.

19. Verfahren nach Anspruch 18, wobei der hydrophobe der Antikrebswirkstoff oder antimikrobielle Wirkstoff oder das Arzneimittel aus Schritt i) vor dem Mischen mit dem Puffer getrocknet wurde.

20. Verfahren nach einem der Ansprüche 18-19, das ferner den Schritt (iii) umfasst, der die Behandlung des in Schritt (ii) erhaltenen Gemischs durch Extrusion oder Beschallung umfasst.

21. Verfahren zur Herstellung des Gels, wie in Anspruch 6 definiert, das mindestens die folgenden Schritte umfasst:
(i) Bereitstellung eines Gemischs, das Folgendes umfasst:
einen wässrigen Puffer;
mindestens einen Antikrebswirkstoff oder einen antimikrobiellen Wirkstoff oder jedes andere Arzneimittel; und das Glycerolipid der Formel (I);
wobei die Konzentration des Glycerolipids der Formel (I) in dem Gemisch höher als 3 mg/ml ist;
ii) Rühren des Gemischs bei einer Temperatur von mehr als 40 ºC,
wobei, wenn der Antikrebswirkstoff oder der antimikrobielle Wirkstoff oder das andere Arzneimittel eine hydrophobe Verbindung ist, der Antikrebswirkstoff oder der antimikrobielle Wirkstoff oder das andere Arzneimittel dem Gemisch mit dem Glycerolipid der Formel (I) hinzugefügt wird, und
wobei, wenn der Antikrebswirkstoff oder der antimikrobielle Wirkstoff oder das andere Arzneimittel eine hydrophile Verbindung ist, der Antikrebswirkstoff oder der antimikrobielle Wirkstoff oder das andere Arzneimittel in den wässrigen Puffer eingearbeitet wird.

## Revendications

1. Liposome comprenant
un noyau et
au moins une bicouche lipidique qui comprend un glycérolipide de formule (I) : 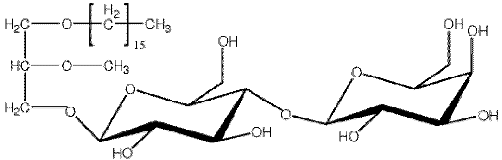
dans lequel le noyau comprend un agent anticancéreux ou un agent antimicrobien hydrophiles ou tout autre médicament hydrophile et/ou
la bicouche lipidique comprend un agent anticancéreux ou un agent antimicrobien hydrophobes ou tout autre médicament hydrophobe.

2. Liposome selon la revendication 1, dans lequel la bicouche lipidique est composée du glycérolipide de formule (I) :

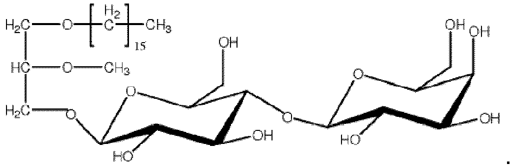

3. Liposome selon l'une quelconque des revendications 1 à 2, dans lequel un agent anticancéreux hydrophile est encapsulé dans le noyau.

4. Liposome selon l'une quelconque des revendications précédentes, dans lequel le liposome comprend un agent anticancéreux et l'agent anticancéreux est choisi dans la liste constituée de : inhibiteurs d'aromatase, anti-œstrogène, anti-androgène, agoniste de la gonadoréline, inhibiteurs de topoisomérase 1, inhibiteurs de topoisomérase 2, agents actifs sur les microtubules, agents alkylants, anthracyclines, corticostéroïdes, IMiD, inhibiteurs de protéase, inhibiteurs d'IGF-1, anticorps CD40, Smac mimétiques, modulateurs du FGF3, inhibiteurs de mTOR, inhibiteurs de HDAC, inhibiteurs d'IKK, inhibiteurs de P38MAPK, inhibiteurs de HSP90, inhibiteurs d'AKT, agents antinéoplasiques, antimétabolites, composés contenant du platine, agents ciblant les lipides ou les protéines kinases, agents ciblant les phosphatases protéiques ou lipidiques, agents anti-angiogéniques, agents qui induisent la différenciation cellulaire, antagonistes du récepteur de bradykinine 1, antagonistes d'angiotensine II, inhibiteurs de cyclo-oxygénase, inhibiteurs d'héparanase, inhibiteurs de lymphokine, inhibiteurs de cytokines, bisphosphanates, inhibiteurs de voie antiapoptotique, agonistes de voie apoptotique, agonistes PPAR, inhibiteurs d'isoformes de Ras, inhibiteurs de télomérase, inhibiteurs de protéase, inhibiteurs de métalloprotéinase et inhibiteurs d'aminopeptidase, agent cytostatique, agent cytotoxique, taxane, antibiotique antitumoral, inhibiteur de topoisomérase II, inhibiteur de topoisomérase I, agent interagissant avec la tubuline, anticorps, antiangiogéniques, inhibiteurs de COX-2, agent hormonal, inhibiteur de thymidilate synthase, anti-métabolite, agent alkylant, inhibiteur de farnésyltransférase, inhibiteur de transduction de signal, inhibiteur de kinase d'EGFR, anticorps anti-EGFR, inhibiteur de kinase C-abl, combinaison de thérapie hormonale, inhibiteur d'aromatase et des combinaisons de ceux-ci.

5. Liposome selon la revendication précédente, dans lequel l'agent anticancéreux est un taxane choisi dans la liste constituée de docétaxel, paclitaxel, tesetaxel, cabazitaxel.

6. Gel d'un glycérolipide de formule (I) : 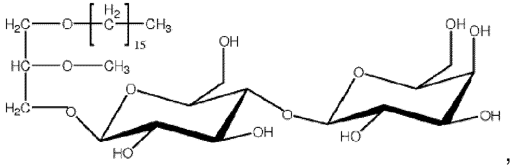 dans lequel les molécules de glycérolipides forment des nanotubes lipidiques.

7. Liposome ou gel selon les revendications précédentes destiné à être utilisé en tant que vecteur d'ADN, d'ARN interférents (siRNA), d'ARN, d'anticorps ou de nanoanticorps.

8. Composition pharmaceutique comprenant un liposome selon l'une quelconque des revendications 1 à 5 ou comprenant le gel selon la revendication 6, et au moins un excipient pharmaceutiquement acceptable et/ou un adjuvant.

9. Composition pharmaceutique selon la revendication précédente dans laquelle l'excipient pharmaceutiquement acceptable est choisi dans la liste constituée d'un stabilisateur ou d'un tensioactif, dans laquelle le stabilisateur ou le tensioactif est un lipide anionique/cationique et/ou le stabilisateur et le tensioactif ont une concentration dans la composition de 5 à 45 % p/p, par rapport au poids total de la composition.

10. Composition pharmaceutique selon l'une quelconque des revendications 8 à 9, dans laquelle la composition est un vaccin.

11. Liposome selon l'une quelconque des revendications 1 à 5 ou gel selon la revendication 6 ou composition pharmaceutique selon l'une quelconque des revendications 8 à 9 destinés à être utilisés en tant que médicament.

12. Liposome ou gel destiné à être utilisé selon la revendication 11, dans lequel le liposome ou le gel comprend un agent antimicrobien et qui est destiné à être utilisé dans le traitement d'une infection bactérienne, virale, protozoaire ou fongique.

13. Liposome ou gel destiné à être utilisé selon la revendication 12, dans lequel l'infection est une infection bactérienne associée à un biofilm.

14. Liposome ou gel destiné à être utilisé selon l'une quelconque des revendications 12 ou 13, dans lequel l'infection est une infection bactérienne causée par *Escherichia coli ou Staphylococcus aureus.*

15. Liposome, gel ou composition pharmaceutique destinée à être utilisée selon la revendication 11, destinée à être utilisée dans le traitement ou la prévention du cancer et/ou la prévention ou la réduction de métastases cancéreuses, dans laquelle le liposome ou le gel comprend un agent anticancéreux.

16. Liposome, gel ou composition pharmaceutique destinée à être utilisée selon la revendication 15, dans laquelle l'utilisation dans le traitement du cancer est destinée à une inhibition ou réduction de la croissance de tumeur primaire.

17. Liposome, gel ou composition pharmaceutique destinée à être utilisée selon la revendication 15, dans laquelle le cancer est choisi dans la liste constituée de : cancer du sein, cancer de la tête et du cou, cancer de l'estomac, cancer de la prostate, cancer du poumon non à petites cellules, leucémie, cancer de la peau, mélanome, cancer du foie, cancer de l'ovaire, cancer du pancréas, cancer du poumon, cancer du rein, cancer du côlon, cancer du cerveau, choisis parmi les gliomes et les glioblastomes, de ceux-ci.

18. Procédé pour la préparation d'un liposome tel que défini dans l'une quelconque des revendications 1 à 5, comprenant au moins les étapes consistant à :
(i) fournir un mélange qui comprend
un tampon aqueux ;
au moins un agent anticancéreux ou un agent antimicrobien ou un quelconque autre médicament ; et le glycérolipide de formule (I) ;
dans lequel la concentration du glycérolipide de formule (I) dans le mélange est inférieure ou égale à 3 mg/ml ;
ii) agiter le mélange à une température supérieure à 40 ºC
dans lequel, lorsque l'agent anticancéreux ou antimicrobien ou l'autre médicament est un composé hydrophobe, l'agent anticancéreux ou antimicrobien ou l'autre médicament est ajouté au mélange avec le glycérolipide de formule (I) obtenu à l'étape précédente et dans lequel, lorsque l'agent anticancéreux ou antimicrobien ou l'autre médicament est un composé hydrophile, l'agent anticancéreux est incorporé dans le tampon aqueux.

19. Procédé selon la revendication 18, dans lequel l'agent anticancéreux ou antimicrobien hydrophobe ou le médicament de l'étape i) a été préalablement séché avant d'être mélangé au tampon.

20. Procédé selon l'une quelconque des revendications 18 à 19, qui comprend en outre l'étape (iii), qui comprend le traitement du mélange obtenu à l'étape ii) par extrusion ou sonication.

21. Procédé pour la préparation du gel tel que défini dans la revendication 6, comprenant au moins les étapes consistant à :
(i) fournir un mélange qui comprend
un tampon aqueux ;
au moins un agent anticancéreux ou un agent antimicrobien ou un quelconque autre médicament ; et le glycérolipide de formule (I) ;
dans lequel la concentration du glycérolipide de formule (I) dans le mélange est supérieure à 3 mg/ml ;
ii) agiter le mélange à une température supérieure à 40 °C,
dans lequel lorsque l'agent anticancéreux ou antimicrobien ou l'autre médicament est un composé hydrophobe, l'agent anticancéreux ou antimicrobien ou l'autre médicament est ajouté au mélange avec le glycérolipide de formule (I) et
dans lequel lorsque l'agent anticancéreux ou antimicrobien ou l'autre médicament est un composé hydrophile, l'agent anticancéreux ou antimicrobien ou l'autre médicament est incorporé dans le tampon aqueux.
